(19) Europäisches Patentamt / European Patent Office / Office européen des brevets

(11) EP 3 256 120 B1

(12) # EUROPEAN PATENT SPECIFICATION

(45) Date of publication and mention of the grant of the patent:
**14.11.2018 Bulletin 2018/46**

(21) Application number: **16720555.8**

(22) Date of filing: **22.03.2016**

(51) Int Cl.:
*A61K 31/37* (2006.01) *A61P 35/00* (2006.01)
*A61P 29/00* (2006.01) *A61P 25/00* (2006.01)
*A61K 31/4025* (2006.01)

(86) International application number:
**PCT/IB2016/051601**

(87) International publication number:
**WO 2016/151483 (29.09.2016 Gazette 2016/39)**

(54) **PSORALEN DERIVATIVES AS NON-PEPTIDIC INHIBITORS OF CHYMOTRYPSIN-LIKE ACTIVITY OF THE IMMUNOPROTEASOME**

PSORALENDERIVATE ALS NICHTPEPTIDISCHE INHIBITOREN DER CHYMOTRYPSINARTIGEN AKTIVITÄT DES IMMUNPROTEASOMS

DÉRIVÉS DU PSORALÈNE UTILISÉS COMME INHIBITEURS NON PEPTIDIQUES DE L'ACTIVITÉ DE TYPE CHYMOTRYPSINE DE L'IMMUNOPROTÉASOME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.03.2015 SI 201500075 P**

(43) Date of publication of application:
**20.12.2017 Bulletin 2017/51**

(73) Proprietor: **Univerza v Ljubljani**
**1000 Ljubljana (SI)**

(72) Inventors:
- **SOSIC, Izidor**
  **1000 Ljubljana (SI)**
- **GOBEC, Martina**
  **1000 Ljubljana (SI)**
- **BRUS, Boris**
  **1235 Radomlje (SI)**
- **KNEZ, Damijan**
  **2367 Vuzenica (SI)**
- **OBREZA, Ales**
  **1295 Ivancna Gorica (SI)**
- **MLINARIC-RASCAN, Irena**
  **1260 Ljubljana (SI)**
- **GOBEC, Stanislav**
  **1000 Ljubljana (SI)**

(74) Representative: **Zacco GmbH**
**Bayerstrasse 83**
**80335 München (DE)**

(56) References cited:
**EP-A1- 0 197 856** **WO-A1-99/26476**
**WO-A1-2008/066255** **WO-A1-2014/006045**
**WO-A2-93/07748**

- **LOURDES SANTANA ET AL: "Quantitative Structure-Activity Relationship and Complex Network Approach to Monoamine Oxidase A and B Inhibitors", JOURNAL OF MEDICINAL CHEMISTRY, vol. 51, no. 21, 13 November 2008 (2008-11-13), pages 6740-6751, XP055280373, US ISSN: 0022-2623, DOI: 10.1021/jm800656v**
- **GIOVANNI MARZARO ET AL: "Psoralen Derivatives as Inhibitors of NF-[kappa]B/DNA Interaction: Synthesis, Molecular Modeling, 3D-QSAR, and Biological Evaluation", JOURNAL OF MEDICINAL CHEMISTRY, vol. 56, no. 5, 14 March 2013 (2013-03-14) , pages 1830-1842, XP055280361, US ISSN: 0022-2623, DOI: 10.1021/jm3009647**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- CHENG T J R ET AL: "High-throughput identification of antibacterials against methicillin-resistant Staphylococcus aureus (MRSA) and the transglycosylase", BIOORGANIC & MEDICINAL CHEMISTRY, PERGAMON, GB, vol. 18, no. 24, 15 December 2010 (2010-12-15), pages 8512-8529, XP027524846, ISSN: 0968-0896 [retrieved on 2010-10-21]
- MARCIANI MAGNO S ET AL: "Interaction of 3-substituted psoralen derivatives with DNA", MEDECINE. BIOLOGIE. ENVIRONNEMENT, PAVIA, IT, vol. 9, no. 1, 1 January 1981 (1981-01-01) , pages 323-334, XP009190527, ISSN: 0302-0800
- TOTH K ET AL: "Characterization of new furocoumarin derivatives by their dark and light-mediated action on RNA bacteriophage MS2", JOURNAL OF PHOTOCHEMISTRY AND PHOTOBIOLOGY B: BIOLOGY, ELSEVIER SCIENCE S.A., BASEL, CH, vol. 2, no. 2, 1 September 1988 (1988-09-01), pages 209-220, XP026531081, ISSN: 1011-1344, DOI: 10.1016/1011-1344(88)80004-6 [retrieved on 1988-09-01]
- FARAG N A H ET AL: "Design, synthesis and docking studies of new furobenzopyranones and pyranobenzopyranones as photoreagent towards DNA and as antimicrobial agents", EUROPEAN JOURNAL OF MEDICINAL CHEMISTRY, EDITIONS SCIENTIFIQUE ELSEVIER, PARIS, FR, vol. 45, no. 1, 1 January 2010 (2010-01-01), pages 317-325, XP026808501, ISSN: 0223-5234 [retrieved on 2009-10-14]
- I V Nagorichna ET AL: "Modified Coumarins. 10. Synthesis of Substituted 2-(7-Oxofuro[3,2-g]chromen-6-yl)acetic Acids", Chemistry of Natural Compounds, 1 May 2003 (2003-05-01), pages 253-261, XP055280538, New York DOI: 10.1023/A:1025466317733 Retrieved from the Internet: URL:http://download.springer.com/static/pdf/583/art%3A10.1023%2FA%3A1025466317733.pd f?originUrl=http://link.springer.com/article/10.1023/A:1025466317733&token2=exp=1465975212~acl=/static/pdf/583/art%253A10.1023%252FA%253A1025466317733.pdf?originUrl=ht t p%3A%2F%2Flink.springer.com%2Farticle%2F10 .1023%2FA% [retrieved on 2016-06-15]
- M. M. Garazd ET AL: "Synthesis of Modified Psoralen Analogues", Russian Journal of Bioorganic Chemistry Original Russian Text Copyright, 1 January 2004 (2004-01-01), pages 291-300, XP055280540, Retrieved from the Internet: URL:http://download.springer.com/static/pdf/786/art%3A10.1023%2FB%3ARUBI.0000030137. 88504.10.pdf?originUrl=http://link.springer.com/article/10.1023/B:RUBI.0000030137.88504.10&token2=exp=1465975456~acl=/static/pdf/786/art%253A10.1023%252FB%253ARUBI.0000 00 030137.88504.10.pdf?originUrl=http%3A%2F%2Flink.spri [retrieved on 2016-06-15]
- ZACHARY MILLER ET AL: "Inhibitors of the Immunoproteasome: Current Status and Future Directions", CURRENT PHARMACEUTICAL DESIGN, vol. 19, no. 22, 1 May 2013 (2013-05-01), pages 4140-4151, XP055280411, NL ISSN: 1381-6128, DOI: 10.2174/1381612811319220018
- Izidor Sosic ET AL: "Nonpeptidic Selectivel Inhibitors of the Chymotrypsin-Like(beta5i) Subunit of the Immunoproteasome", , 1 April 2016 (2016-04-01), pages 5745-5748, XP055280308, Retrieved from the Internet: URL:http://onlinelibrary.wiley.com/store/10.1002/anie.201600190/asset/anie201600190.pdf?v=1&t=ipfcmhwh&s=8c616be35624408512159 8c40cc004cb2e68e572 [retrieved on 2016-06-14]

## Description

### Technical Field

The present invention belongs to the field of pharmaceutical industry and relates to novel inhibitors of chymotrypsin-like activity of the immunoproteasome (inhibitors of β5i or LMP7 subunit) with a psoralen-based structure in the form of pure diastereoisomers, mixture of diastereomers, in the form of pure enantiomers, mixture of enantiomers, or pharmaceutically acceptable salts, hydrates or solvates thereof. Such compounds are particularly useful for the treatment of diseases that are related with increased activity of the immunoproteasome. The present invention also relates to the preparation of these compounds, use of these compounds, and to the pharmaceutical products that contain these compounds. The characteristic of these compounds is that they bind into the active site of the β5i subunit through specific noncovalent (polar and hydrophobic interactions) or concurrent noncovalent (polar and hydrophobic interactions) and covalent interactions.

### Background Art

The proteasome is a large (2.5 MDa) ATP-dependent proteolytic complex that is present in every eukaryotic cell, in archaea, and in some bacteria. It represents the key component of the ubiquitin-proteasome system as it is responsible for the degradation of the majority of proteins into peptides in the cytosol and in the nucleus of the cells. It consists of a 20S core particle with 19S regulatory particles attached to one or both ends of the core. The 20S cores (approximately 700 kDa) are hollow cylindrical structures comprising two pairs of 14 different polypeptides arranged in four stacked rings, two inner β rings and two outer α rings (Kish-Trier E. et al. Ann. Rev. Biophys. 2013, 49, 29-49). Both inner rings contain seven subunits (β1 - β7), whereas the proteolytic activity resides within three catalytic subunits (one copy in each β-ring), namely β1 (also referred to as caspase-like, cleaving preferentially after acidic residues), β2 (trypsin-like, with a preference for basic residues), and β5 (chymotrypsin-like, preferring neutral, hydrophobic residues at the cleavage site). Of note, the proteasome utilizes the N-terminal Thr residues of the catalytic subunits to carry out the hydrolysis of the peptide bond (Arendt C.S. et al. Proc. Natl. Acad. Sci. 1997, 94, 7156-7161).

While the constitutive proteasome is expressed in all cell types, the immunoproteasome is predominantly expressed in cells of hematopoietic origin (e.g. monocytes and lymphocytes). However, during inflammation, cytokines such as interferon-γ and tumor necrosis factor (TNF) α also induce the formation of the immunoproteasome in cells of non-hematopoietic origin. The immunoproteasome is formed by the replacement of the constitutive catalytic subunits β, β2, and β5 with the immunoproteasome-specific catalytic subunits β1i (LMP2, low molecular weight protein 2, PSMB9), β2i (MECL1, multicatalytic endopeptidase complex-like 1, PSMB10), and β5i (LMP7, PSMB8) (Groettrup M. et al. Nat. Rev. Immunol. 2010, 10, 73-78). Upregulated expression and increased activity of the immunoproteasome are characteristic for various diseases that are described in more details in the next paragraphs.

AUTOIMMUNE DISEASES

As expected form the regulatory roles of proinflammatory cytokines in the formation of the immunoproteasome, the upregulation of this protease has been reported in a number of autoimmune disorders, as well as inflammatory diseases (Lee W. et. al. Curr. Med. Chem. 2011, 11, 2923-2930). One such example is the primary Sjogren syndrome, a chronic autoimmune disease, in which the exocrine glands (specifically the salivary and lacrimal glands) are affected. The studies show marked upregulation of the β1i, β2i, β5i, and PA28α at the mRNA level in the peripheral blood mononuclear cells and salivary glands from the patients with the primary Sjogren syndrome (Krause S. et al. Ann. Rheum. Dis. 2006, 65, 1021-1027). Elevated levels of the immunoproteasome subunits have been reported also in various chronic inflammatory bowel diseases, such as Chron's disease and ulcerative colitis (Basler M. et al. J. Immunol. 2010, 185, 634-641; Visekruna A. et. al. J. Clin. Invest. 2006, 116, 3195-320; Schmidt N. et. al. Gut 2010, 59, 896-906). Furthermore, the β1i subunit was found to be upregulated in a mouse model of dextrane sulfate induced colitis. In the same model, it was also shown that the β5i-selective inhibitor PR-957 reduces the production of various proinflammatory cytokines and thus effectively reduces the inflammation and mitigates damages to colon mucosa (Basler M. et al. J. Immunol. 2010, 185, 634-641). The importance of the β5i subunit in the colitis development was additionally confirmed with β5i-deficient mice that showed attenuated disease progression in comparison with wild-type mice (Schmidt N. et. al. Gut 2010, 59, 896-906). Using mouse models of rheumatoid arthritis and transgenic mice lacking the β5i subunit it was also shown that the immunoproteasome plays a key role in the pathogenic immune response. Importantly, PR-957 attenuated various inflammatory responses (lowering the production of interleukin (IL)-6, IL-23, and TNFα; reduction of the expression of MHC-I receptors on the surface to 50%) and reduced the severity of disease symptoms (Muchamuel T. et. al. Nat. Med. 2009, 15, 781-787).

MALIGNANT DISEASES

Cancer cells are generally associated with increased proteasome activity compared to non-malignant cells, possibly related to the rapid cell proliferation, increased oxidative stress, and elevated cytokine levels. Because of the importance and involvement of proteasome in above-mentioned biological processes, it has been thoroughly investigated as a target for the treatment of malignant diseases (Frankland-Searby S. et. al. Biochim. Biophys. Acta 2012, 1825, 64-76). While the activity of the constitutive proteasome is generally found to be upregulated in the majority of cancers, the levels of the immunoproteasome in cancer cells are either upregulated (multiple myeloma, prostate cancer, colon cancer, lung cancer) or, in some cases, downregulated (esophageal, renal, skin, neck cancers) (Lee W. et. al. Curr. Med. Chem. 2011, 11, 2923-2930). Multiple myeloma (MM) is a consequence of malignantly transformed plasma cells, a subtype of lymphocytes B. The MM is treated with various immunomodulatory drugs, steroids, and proteasome inhibitors, such as bortezomib. Bortezomib is a dipeptide boronate that inhibits the catalytic activities of both the constitutive proteasome and the immunoproteasome. Despite its success on the market, it has several disadvantages, such as thrombocytopenia and neutropenia. This is most probably the result of the inhibition of constitutive proteasome in healthy cells. Therefore, selective inhibition of the immunoproteasome should represent improved strategy for the treatment of MM, as it should lead to improved toxicity profile over broadly-acting proteasome inhibitors and fewer side effects (Bellavista E. et. al. Curr. Pharm. Design 2013, 19, 702-718). It has been shown that the β1i-selective inhibitor IPSI-001 induces apoptosis in haematological malignancies, which express elevated immunoproteasome levels, as well as in bortezomib-resistant cells, and appears to be less cytotoxic to nonmalignant cells (Kuhn D.J. et. al. Blood 2009, 113, 4667-4676). Furthermore, the selective inhibitor of β5i subunit, PR-924, was shown to induce apoptosis in numerous MM cell lines and strong antitumor activity in two xenograft models of MM. Concurrently, no significant toxicities were observed in normal peripheral blood mononuclear cells following exposure to high (up to 20 μM) conentrations of PR-924 (Singh A.V. et. al. Br. J. Haematol. 2011, 152, 155-163).

NEURODEGENERATIVE DISEASES

Neurodegenerative diseases, such as Alzheimer's disease, amyotrophic lateral sclerosis and Huntington's disease have been frequently associated with accumulation of protein aggregates due to an imbalance between protein synthesis and proteolysis. While the overall activity of the ubiquitin-proteasome system has been shown to be substantially decreased in many of the above-mentioned neurodegenerative diseases, several research groups have shown that the levels of the immunoproteasome-specific subunits are upregulated in these disease settings. For example, upregulation of the β1i i and β5i subunits has been observed in animal models of Huntington's disease (Diaz-Hernandez M. et. al. J. Neurosci. 2003, 23, 11653-11661). However, it must be noted that the immunoproteasome subunits in the human brain tissues show an age-related upregulation, which may also be associated with chronic inflammation of the aged brain (Mishto M. et. al. Neurobiol. Aging 2006, 27, 54-66).

INHIBITORS OF THE IMMUNOPROTEASOME

The majority of currently known inhibitors are small, peptide-like compounds that mimic the binding mode of natural substrates of the immunoproteasome through the formation of an antiparallel β sheet in the substrate binding channel of the active site cleft. In addition to peptidic backbone, these compounds are endowed with a reactive functional group (warhead) in the structure that leads to improved inhibitory activity and higher specificity. This reactive warhead modifies either reversibly or irreversibly the Thr1 residue of active proteasomal β subunits by formation of a covalent bond (Huber E.M. et. al. Angew. Chem. Int. Ed. 2012, 51, 2-15).

As the field of immunoproteasome biology advances and because its role in various diseases has become evident, the development of immunoproteasome-specific inhibitors has become the focus of recent studies. In particular, current results indicate that selective inhibition of β1i and β5i subunits is of special interest (Miller Z. et. al. Curr. Pharm. Design. 2013, 19, 4140-4151). The most advanced β5i-selective inhibitors are PR-957 and PR-924. The former is an irreversibly acting tripeptide α',β'-epoxyketone that has a promising potential for the treatment of inflammatory and autoimmune diseases. Targeted inhibition of the β5i subunit in mouse models of rheumatoid arthritis was found to offer significant therapeutic benefits, as it blocked disease progression and alleviated the severity of disease symptoms. PR-957 also inhibited the production of proinflammatory cytokines such as IL-23 and TNFα in activated monocytes (Muchamuel T. et. al. Nat. Med. 2009, 15, 781-787). Recently, PR-957 was investigated as a potential therapy for the treatment of systemic lupus erythematosus, a complex autoimmune disease that can affect multiple organs in the body (such as blood cells, skin, joints, kidneys). When tested using lupus-prone mice, the β5i-selective PR-957 was as highly effective as bortezomib or carfilzomib that inhibit both the constitutive proteasome and the immunoproteasome. The fact that all inhibitors prevent disease progression and reduce the symptoms of nephritis to a similar extent, indicate the major advantage of PR-957, i.e. potentially less systemic toxicity as a result of the β5i-selective mechanism of action

(Ichikawa H.T. et. al. Arthritis Rheum. 2012, 64, 493-503). PR-924 is another epoxyketone peptide-based inhibitor with structural similarities to PR-957. The selectivity of PR-924 for inhibition of the β5i ($IC_{50}$ = 22 nM) was shown to be greater that 100-fold over proteasomal β5 and little or no inhibition was observed for other catalytic subunits (β1, β1i, β2, β2i). As mentioned above, it was shown in a recent study that PR-924 can effectively cause cell death in both clinical samples from MM patients an in numerous MM cell lines (Singh A.V. et. al. Br. J. Haematol. 2011, 152, 155-163).

## Summary of Invention

The invention relates to novel inhibitors of the chymotrypsin-like activity of the immunoproteasome with the general Formula (I), with A-E, X and Z as defined in the appended claims, where substituents are clearly defined in the form of pure enantiomers, mixture of enantiomers, in the form of pure diastereoisomers, mixture of diastereomers, and their pharmaceutically acceptable salts, hydrates or solvates. The invention relates to the use of the described compounds for the treatment of diseases where immunoproteasome activity is increased.

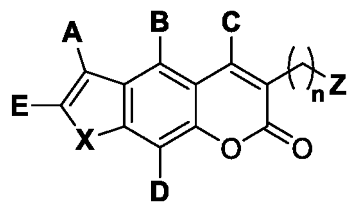

**(I)**

## Technical Problem

New medicines for the treatment of autoimmune diseases, malignant diseases, and neurodegenerative disorders are urgently needed. The current arrays of compounds that act as inhibitors of the β5i subunit and are used in this research field are of peptidic nature. Such compounds have low bioavailability and are prone to nonselective degradation by various proteases. The subject of the present invention is the discovery of compounds with non-peptidic structure that bind into the active site of the β5i (LMP7) subunit of the immunoproteasome and inhibit its activity.

## Solution to Problem

The present invention relates to a compound of Formula (I) [0017]

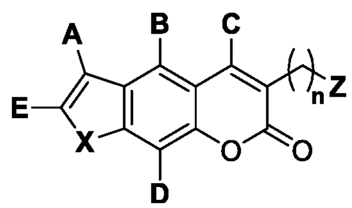

**(I)**

wherein:

**n**: 0-4

**X**: O

**A:** is t-Bu, phenyl, furan, thiophene, pyrrole, imidazole, oxazole,

**B**: is H or Me

**C**: is H or Me

**D**: is H or Me

**E**: is H or Me

**Z**: is CHO, $CONHCH_2CN$, $CON(Me)CH_2CN$, $NHCOCH_2CN$, $N(Me)COCH_2CN$, $NHCH_2CN$, $N(Me)CH_2CN$, NHCO-COMe, N(Me)C(=O)COMe, $NHCOCH=CH_2$, $N(Me)COCH=CH_2$, $SO_2F$,

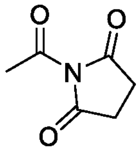, 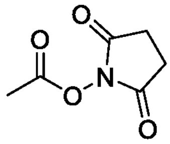, 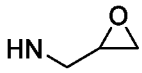, 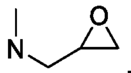, 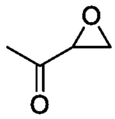, 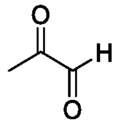, 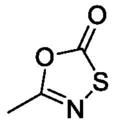

Some compounds from this invention possess stereogenic center with an absolute configuration of R or S, where the compounds can appear in a racemic form, in a form of conglomerate or in a form of pure enantiomers.

The invention further relates to the pharmaceutically acceptable salts with the general Formula (I).

The invention further relates to the use of the compounds with the general Formula (I) as active pharmaceutical ingredients for the preparation of medicaments. Compounds with the general Formula (I) are inhibitors of the β5i subunit of the immunoproteasome and are used for the treatment of diseases that are related with increased activity of the immunoproteasome.

The invention is related to parenteral, peroral, or other pharmaceutically acceptable forms containing the compounds with the general Formula (I). Beside active pharmaceutical ingredient, the pharmaceutical composition can contain excipients suitable for the intended route of administration. Pharmaceutical compositions are prepared using standard procedures. Pharmaceutical compositions can be prepared in the way that ensures the sustained release of the active pharmaceutical ingredient. The dose, frequency, and way of use depend on several factors, which further depend on the active pharmaceutical ingredient used, its pharmacokinetic properties and the condition of the patient.

## Examples

### General synthetic procedures

### Procedure A (*O*-alkylation)

To a round-bottomed flask, a starting 7-hydroxycoumarine derivative (ethyl 3-(7-hydroxy-4-methyl-2-oxo-2*H*-chromen-3-yl)propanoate; ethyl 3-(7-hydroxy-4-methyl-2-oxo-2*H*-chromen-3-yl)acetate; ethyl 3-(7- hydroxy -2-oxo-2*H*-chromen - 3-yl)acetate; ethyl 7-hydroxy-4-methyl-2-oxo-2*H*-chromen-3-carboxylate; *N*-(7-hydroxy-4-methyl-2-oxo-2*H*-chromen-3-yl)acetamide) (0.5-5.0 g, 1.0 mol. equivalent), a suitable commercially available or according to the literature prepared (US 6346534 B1) α-haloketone (1.5 mol. equivalent), $K_2CO_3$ (4.0 mol. equivalent) and KI (0.1 mol. equivalent) were added. Then, 1,4-dioxane (20-50 mL) was added and the mixture was stirred for 24 h at 100 °C. The solvent was evaporated under reduced pressure, EtOAc (150 mL) and water (150 mL) were added to the resulting residue, and transferred to a separating funnel where layers were separated. The aqueous layer was further extracted with EtOAc (3 × 100 mL). The combined organic phases were washed with saturated aqueous solution of $NaHCO_3$ (100 mL), saturated brine (100 mL), dried over $Na_2SO_4$, filtered, and evaporated under reduced pressure. The crude products were subsequently purified by crystallization or column chromatography.

### Procedure B (Base-catalyzed cyclization)

To the starting compound (0.1-5.0 g, 1.0 mol. equivalent), 1 M NaOH (6.0-10.0 mol. equivalent) and propan-2-ol (10-20 mL) were added. The reaction mixture was stirred for 3 h at 100 °C, then cooled to room temperature, and acidified with 15% HCl to pH 1-2. The mixture was transferred to a separating funnel using EtOAc (150 mL) where the layers were separated. The aqueous layer was further extracted with EtOAc (3 × 50 mL). The combined organic phases were dried over $Na_2SO_4$, filtered, and evaporated under reduced pressure. The crude products were subsequently purified by crystallization or column chromatography.

### Procedure C (Synthesis of *N*-(cyanomethyl)amides)

To the starting compound (carboxyalkyl psoralen, 0.05-0.50 g, 1.0 mol. equivalent), *N*-methylaminoacetonitrile hydrochloride or aminoacetonitrile hydrochloride (1.25 mol. equivalent), THF (10-15 mL), EDC (1.25 mol. equivalent), and $Et_3N$ (1.25 mol. equivalent) were added under argon atmosphere. The resulting suspension was stirred at room

temperature for 24 h. Then, the solvent was evaporated under reduced pressure. To the resulting residue, 1 M HCl (20-50 mL) and EtOAc (20-50 mL) were added, transferred to a separating funnel, shaken intensively and left to allow the separation of the layers. The aqueous layer was further extracted with EtOAc (4 × 20-70 mL). The combined organic phases were dried over $Na_2SO_4$, filtered, and evaporated under reduced pressure. The crude products were subsequently purified by crystallization or column chromatography.

EXAMPLE 1

**Synthesis of 3-(5-methyl-7-oxo-3-phenyl-7*H*-furo[3,2-*g*]chromen-6-yl)propanoic acid**

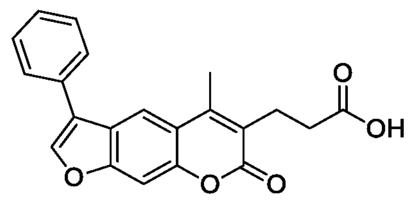

*Step 1:* **Ethyl 3-(7-hydroxy-4-methyl-2-oxo-2*H*-chromen-3-yl)propanoate**

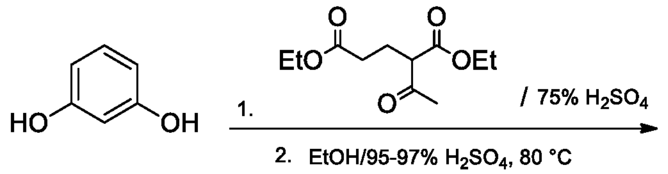


To the solution of resorcinol (5.00 g, 45.41 mmol) in sulfuric (VI) acid (75%, 60 mL), diethyl 2-acetylglutarate (10.56 g, 45.86 mmol, 9.86 mL) was added dropwise. The reaction mixture was stirred at room temperature for 24 h. After the reaction was complete, the solution was poured onto crushed ice. The solid that formed was filtered off, washed with water and dried. Anhydrous ethanol (60 mL) and the catalytic amount of concentrated sulfuric (VI) acid (0.50 mL) were added to the dried solid. The reaction mixture was stirred at 80 °C overnight. Then, the solvent was evaporated, saturated aqueous solution of $NaHCO_3$ (50 mL) was added to the resulting residue, and the mixture extracted with EtOAc (2 × 100 mL). The combined organic phases were dried over $Na_2SO_4$, filtered, and evaporated under reduced pressure.

**Appearance:** off-white amorphous solid, m = 10.98 g; **Yield:** 88%; **Melting point:** 104-108 °C; **ESI-MS [M+H]$^+$:** 277; **HRMS:** (m/z = 277) for $C_{15}H_{17}O_5$: calculated 277.2846, found 277.2850; **$^1$H NMR (400 MHz, DMSO-*d$_6$*)** δ **(ppm)** = 1.15 (t, *J* = 7.2 Hz, 3H, $CH_2CH_3$), 2.37 (s, 3H, Ar-$CH_3$), 2.46 (t, *J* = 7.5 Hz, 2H, $CH_2CH_2COO$), 2.79 (t, *J* = 7.5 Hz, 2H, $CH_2CH_2COO$), 4.03 (q, *J* = 7.2 Hz, 2H, $CH_2CH_3$), 6.68 (d, *J* = 2.4 Hz, 1H, Ar-H), 6.79 (dd, $J_1$ = 8.8 Hz, $J_2$ = 2.4 Hz, 1H, Ar-H), 7.61 (d, *J* = 8.8 Hz, 1H, Ar-H), 10.43 (bs, 1H, OH); **IR (ATR):** 3240, 2978, 1729, 1670, 1613, 1566, 1293, 1237, 1178, 1154, 1095, 1053, 867, 818, 781, 690

*Step 2:* **Ethyl 3-(4-methyl-2-oxo-7-(2-oxo-2-phenylethoxy)-2*H*-chromen-3-yl)propanoate**

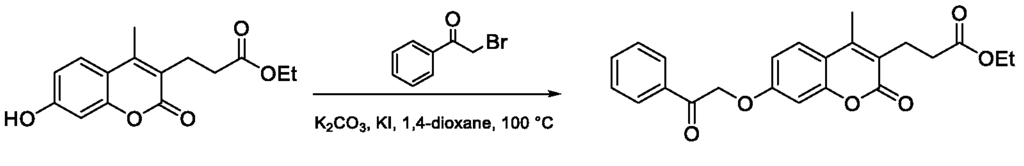


The title compound was prepared from ethyl 3-(7-hydroxy-4-methyl-2-oxo-2*H*-chromen-3-yl)propanoate (1.00 g) and 2-bromo-1-phenylethan-1-one according to the general procedure A (*O*-alkylation). The compound was purified by column chromatography (MP = EtOAc : n-hexane = 1 : 1).

**Appearance:** white amorphous soild, m = 0.56 g; **Yield:** 39%; **Melting point:** 138-142 °C; **ESI-MS [M+H]$^+$:** 395; **$^1$H NMR (400 MHz, DMSO-*d$_6$*) δ (ppm)** = 1.15 (t, *J* = 7.2 Hz, 3H, $CH_2CH_3$), 2.41 (s, 3H, Ar-$CH_3$), 2.48 (t, *J* = 8.0 Hz, 2H, $CH_2CH_2COO$), 2.81 (t, *J* = 8.0 Hz, 2H, $CH_2CH_2COO$), 4.04 (q, *J* = 7.2 Hz, 2H, $CH_2CH_3$), 5.74 (s, 2H, $COCH_2O$), 7.02 (dd, $J_1$ = 8.8 Hz, $J_2$ = 2.5 Hz, 1H, Ar-H), 7.06 (d, *J* = 2.5 Hz, 1H, Ar-H), 7.56-7.61 (m, 2H, Ar-H), 7.69-7.75 (m, 2H, Ar-H), 8.02-8.06 (m, 2H, Ar-H); **IR (ATR):** 2984, 2160, 1974, 1712, 1694, 1612, 1424, 1375, 1296, 1197, 1160, 1092, 998, 976, 841, 760, 689, 641

*Step 3:* **3-(5-Methyl-7-oxo-3-phenyl-7*H*-furo[3,2-*g*]chromen-6-yl)propanoic acid**

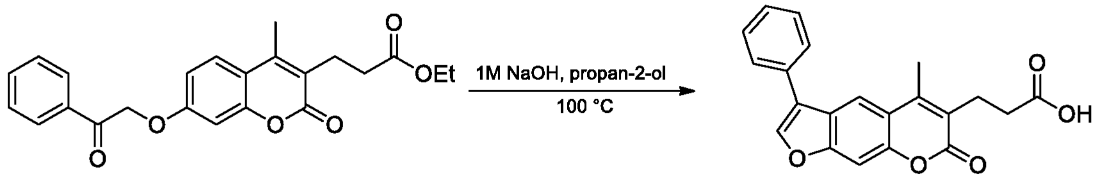


The title compound was prepared from ethyl 3-(4-methyl-2-oxo-7-(2-oxo-2-phenylethoxy)-2*H*-chromen-3-yl)propanoate (0.55 g) following the general procedure B (base-catalyzed cyclization). The compound was purified by crystallization from EtOAc.

**Appearance:** white crystals, m = 0.48 g; **Yield:** 98%; **Melting point** = 189-193 °C; **ESI-MS [M+H]$^+$:** 349; **HRMS:** (m/z = 349) for $C_{21}H_{17}O_5$: calculate 349.1076, found 349.1079; **$^1$H NMR (400 MHz, DMSO-*d$_6$*) δ (ppm)** = 2.44 (t, *J* = 8.0 Hz, 2H, $CH_2CH_2COOH$), 2.55 (s, 3H, Ar-$CH_3$), 2.85 (t, *J* = 8.0 Hz, 2H, $CH_2CH_2COOH$), 7.40-7.46 (m, 1H, Ar-H), 7.51-7.58 (m, 2H, Ar-H), 7.78 (s, 1H, Ar-H), 7.80-7.85 (m, 2H, Ar-H), 8.17 (s, 1H, Ar-H), 8.47 (s, 1H, Ar-H), 12.23 (bs, 1H, COOH); **$^{13}$C NMR (100 MHz, DMSO-*d$_6$*) δ (ppm)** = 15.11, 22.96, 32.28, 99.37, 116.39, 116.88, 121.18, 122.38, 122.70, 127.14, 127.76, 129.15, 130.64, 144.21, 147.86, 149.74, 155.76, 160.44, 173.59; **IR (ATR):** 3056, 2160, 2014, 1738, 1659, 1619, 1572, 1386, 1354, 1297, 1198, 1164, 1088, 840, 760, 687

EXAMPLE 2

**Synthesis of 2-(3-(4-bromophenyl)-5-methyl-7-oxo-7*H*-furo[3,2-*g*]chromen-6-yl)acetic acid**

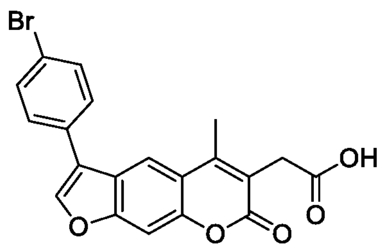

*Step 1:* **Ethyl 2-(7-hydroxy-4-methyl-2-oxo-2H-chromen-3-yl)acetate**

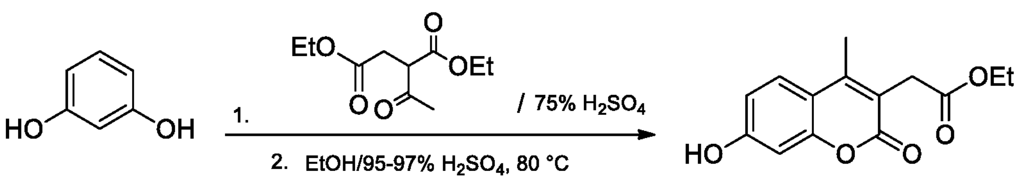


To the solution of resorcinol (5.00 g, 45.41 mmol) in sulfuric (VI) acid (75%, 60 mL), diethyl 2-acetylsuccinate (9.92 g, 45.86 mmol, 9.17 mL) was added dropwise. The reaction mixture was stirred at room temperature for 24 h. After the reaction was complete, the solution was poured onto crushed ice. The solid that formed was filtered off, washed

with water and dried. Anhydrous ethanol (30 mL) and the catalytic amount of concentrated sulfuric (VI) acid (0.10 mL) were added to the dried solid. The reaction mixture was stirred at 80 °C overnight. Then, 1 M NaOH (15 mL) and water (200 mL) were added to the mixture. The precipitate formed that was filtered off and dried overnight at 50 °C.

**Appearance:** white amorphous solid, m = 8.96 g; **Yield:** 75%; **Melting point:** 128-133 °C; **ESI-MS $[M+H]^+$:** 263; **$^1H$ NMR (400 MHz, DMSO-*$d_6$*)** $\delta$ **(ppm)** = 1.18 (t, *J* = 7.2 Hz, 3H, $CH_2CH_3$), 2.35 (s, 3H, Ar-$CH_3$), 3.63 (s, 2H, $CH_2COO$), 4.08 (q, *J* = 7.2 Hz, 2H, $COOCH_2$), 6.72 (d, *J* = 2.5 Hz, 1H, Ar-H), 6.82 (dd, $J_1$ = 8.7 Hz, $J_2$ = 2.5 Hz, 1H, Ar-H), 7.67 (d, *J* = 8.7 Hz, 1H, Ar-H), 10.53 (s, 1H, OH); **IR (ATR):** 3129, 2937, 1720, 1667, 1577, 1444, 1364, 1326, 1226, 1185, 1161, 1140, 1096, 1025, 841, 793, 754

*Step 2:* **Ethyl 2-(7-(2-(4-bromophenyl)-2-oxoethoxy)-4-methyl-2-oxo-2H-chromen-3-yl)acetate**

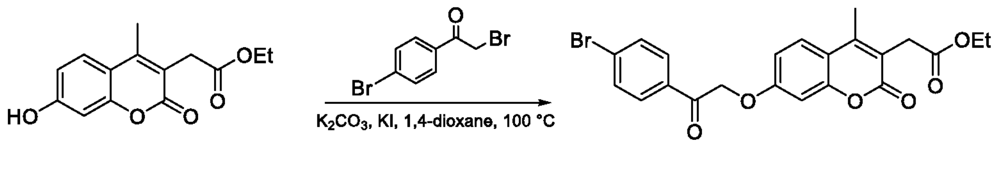


The title compound was prepared from ethyl 3-(7-hydroxy-4-methyl-2-oxo-2*H*-chromen-3-yl)acetate (1.69 g) and 2-bromo-1-(4-bromophenyl)ethan-1-one according to the general procedure A (*O*-alkylation). The compound was purified by column chromatography (MP = EtOAc : n-hexane = 1 : 1).

**Appearance:** orange amorphous solid, m = 0.65 g; **Yield:** 22%; **Melting point:** 157-160 °C; **ESI-MS $[M+H]^+$:** m/z = 459; **HRMS:** (m/z = 459) for $C_{22}H_{20}BrO_6$: calculated 459.0430, found 459.0452; **$^1H$ NMR (400 MHz, DMSO-*$d_6$*)** $\delta$ **(ppm)** = 1.18 (t, *J* = 7.0 Hz, 3H, $CH_2CH_3$), 2.84 (s, 3H, Ar-$CH_3$), 3.66 (s, 2H, $CH_2COO$), 4.08 (q, *J* = 7.0 Hz, 2H, $COOCH_2CH_3$), 5.73 (s, 2H, $COCH_2O$), 7.05 (dd, $J_1$ =8.9 Hz, $J_2$ = 2.6 Hz, 1H, Ar-H), 7.12 (d, *J* = 2.6 Hz, 1H, Ar-H), 7.76 (d, *J* = 8.9 Hz, 1H, Ar-H), 7.79-7.84 (m, 2H, Ar-H), 7.94-8.00 (m, 2H, Ar-H); **IR (ATR):** 3650, 2520, 2160, 2030, 1976, 1696, 1611, 1585, 1327, 1227, 1177, 1095, 1071, 989, 970, 822

*Step 3:* **2-(3-(4-Bromophenyl)-5-methyl-7-oxo-7*H*-furo[3,2-*g*]chromen-6-yl)acetic acid**

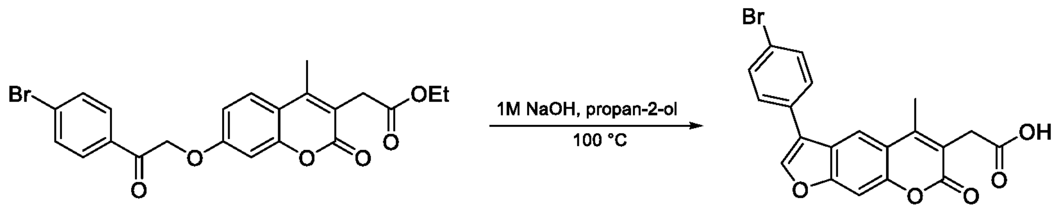


The title compound was prepared from 2-(7-(2-(4-bromophenyl)-2-oxoethoxy)-4-methyl-2-oxo-2*H*-chromen-3-yl)acetate (0.64 g) following the general procedure B (base-catalyzed cyclization). The compound was purified by crystallization from EtOAc.

**Appearance:** white crystals, m = 0.54 g; **Yield:** 95%; **Melting point** 230-235 °C; **ESI-MS $[M+H]^+$:** m/z = 413; **HRMS:** (m/z = 413) for $C_{20}H_{14}BrO_5$: calculated 413.0025, found 413.0023; **$^1H$ NMR (400 MHz, DMSO-*$d_6$*)** $\delta$ **(ppm)** = 2.53 (s, 3H, Ar-$CH_3$), 3.65 (s, 2H, $CH_2COOH$), 7.69-7.75 (m, 2H, Ar-H), 7.76-7.81 (m, 3H, Ar-H), 8.17 (s, 1H, Ar-H), 8.52 (s, 1H, Ar-H), 12.51 (bs, 1H, COOH); $^{13}C$ **NMR (100 MHz, DMSO-*$d_6$*)** $\delta$ **(ppm)** = 15.56, 32.88, 99.58, 116.75, 116.81, 118.27, 120.22, 120.86, 122.49, 129.25, 129.88, 132.04, 144.72, 149.29, 149.89, 155.95, 160.64, 171.43; **IR (ATR):** 3137, 2161, 1753, 1656, 1625, 1573, 1393, 1343, 1296, 1165, 1095, 1076, 862, 780, 771, 749, 706, 629

EXAMPLE 3

**Synthesis of 3-(3-(4-methoxyphenyl)-5-methyl-7-oxo-7H-furo[3,2-*g*]chromen-6-yl)propanoic acid**

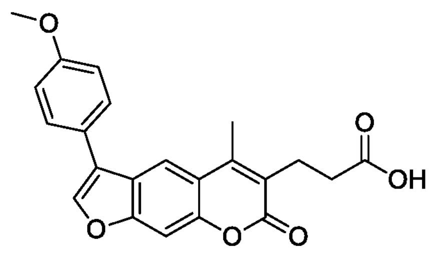

*Step 1:* **Ethyl 3-(7-(2-(4-methoxyphenyl)-2-oxoethoxy)-4-methyl-2-oxo-2*H*-chromen-3-yl)propanoate**

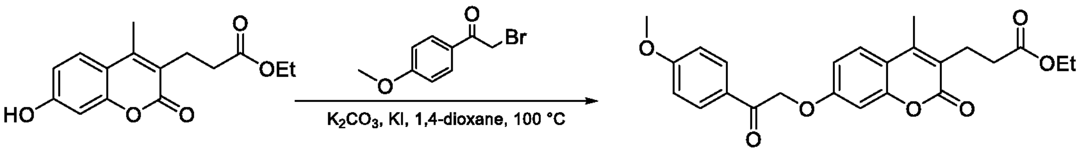


The title compound was prepared from ethyl 3-(7-hydroxy-4-methyl-2-oxo-2*H*-chromen-3-yl)propanoate (0.83 g) and 2-bromo-1-(4-methoxyphenyl)ethan-1-one according to the general procedure A (*O*-alkylation). The compound was purified by column chromatography (MP = $CH_2Cl_2$ : MeOH = 40 : 1).

**Appearance:** brown solid, m = 0.41 g; **Yield:** 32%; **Melting point:** 112-116 °C; **ESI-MS [M+H]$^+$:** m/z = 425; **HRMS:** (m/z = 425) for $C_{24}H_{25}O_7$: calculated 425.1600, found 425.1596; **$^1$H NMR (400 MHz, DMSO-*d$_6$*)** δ **(ppm)** = 1.16 (t, *J* = 7.0 Hz, 3H, $CH_2CH_3$), 2.40 (s, 3H, Ar-$CH_3$), 2.47 (t, *J* = 7.5 Hz, 2H, $CH_2CH_2COO$), 2.81 (t, *J* = 7.5 Hz, 2H, $CH_2CH_2COO$), 3.87 (s, 3H, $OCH_3$), 4.03 (q, *J* = 7.0 Hz, 2H, $CH_2CH_3$), 5.66 (s, 2H, $COCH_2O$), 7.00 (dd, $J_1$ = 8.5 Hz, $J_2$ = 2.5 Hz, 1H, Ar-H), 7.02 (d, *J* = 2.5 Hz, 1H, Ar-H), 7.08-7.12 (m, 2H, Ar-H), 7.71 (d, *J* = 8.5 Hz, 1H, Ar-H), 7.99-8.03 (m, 2H, Ar-H); **IR (KBr):** 2932, 2839, 1728, 1695, 1601, 1508, 1423, 1377, 1330, 1298, 1238, 1167, 1095

*Step 2:* **3-(3-(4-Methoxyphenyl)-5-methyl-7-oxo-7*H*-furo[3,2-*g*]chromen-6-yl)propanoic acid**

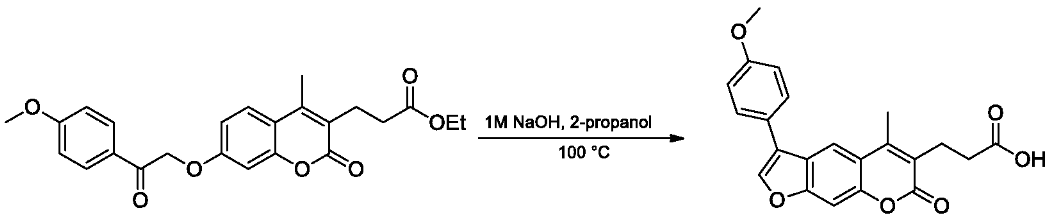


The title compound was prepared from ethyl 3-(7-(2-(4-methoxyphenyl)-2-oxoethoxy)-4-methyl-2-oxo-2*H*-chromen-3-yl)propanoate (0.80 g) following the general procedure B (base-catalyzed cyclization). The compound was purified by crystallization from EtOAc.

**Appearance:** brown crystals, m = 0.63 g; **Yield:** 89%; **Melting point:** 210-214 °C; **ESI-MS [M+H]$^+$:** 379; **HRMS:** (m/z = 379) for $C_{22}H_{19}O_6$: calculated 379.1182, found 379.1190; **$^1$H NMR (400 MHz, DMSO-*d$_6$*)** δ **(ppm)** = 2.43 (t, *J* = 7.5 Hz, 2H, $CH_2CH_2COOH$), 2.56 (s, 3H, Ar-$CH_3$), 2.85 (t, *J* = 7.5 Hz, 2H, $CH_2CH_2COO$), 3.83 (s, 3H, $OCH_3$), 7.07-7.12 (m, 2H, Ar-H), 7.72-7.77 (m, 3H, Ar-H), 8.13 (s, 1H, Ar-H), 8.37 (s, 1H, Ar-H), 12.23 (bs, 1H, COOH); **$^{13}$C NMR (100 MHz, DMSO-*d$_6$*)** δ **(ppm)** = 15.11, 22.96, 32.29, 55.14, 99.30, 113.74, 114.59, 116.35, 116.77, 120.86, 122.31, 122.86, 128.42, 143.33, 147.89, 149.70, 155.71, 158.90, 160.48, 173.60; **IR (ATR):** 2927, 1256, 1162, 1027, 843

EXAMPLE 4

**Synthesis of 2-(7-oxo-3-phenyl-7*H*-furo[3,2-*g*]chromen-6-yl)acetic acid**

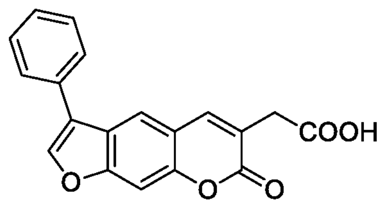

*Step 1:* **Ethyl 2-(7-hydroxy-2-oxo-2H-chromen-3-yl)acetate**

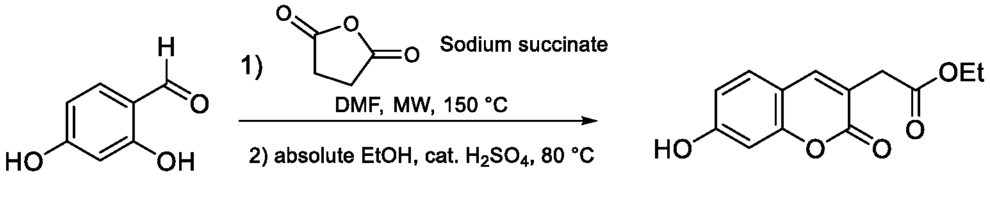


The title compound was prepared as described in the literature (Kovačová S. et al. Chem. Pap. 2010, 64,806-811), with subsequent acid-catalyzed esterification. 2,4-Dihydroxybenzaldehyde (0.83 g, 6.00 mmol), succinic anhydride (3.09 g, 30 mmol) and sodium succinate (0.44 g, 7.98 mmol) in DMF (2.4 mL) were put into reaction vessel and heated with a heat gun until dissolution. The reaction mixture was irradiated in a focused microwave at 150 °C (Discover, CEM Corporation, Matthews, NC, USA) for 15 min and then transferred to a 50 mL round-bottomed flask. Water (10 mL) and concentrated HCl (4 mL) were added, and the resulting solution was stirred at room temperature for 30 min. The solution was made alkaline (pH 9-10) using $K_2CO_3$ and extracted with $CH_2Cl_2$ (2 × 30 mL). The organic layer was discharged. The aqueous layer was acidified with concentrated HCl and extracted with EtOAc (3 × 50 mL). The combined organic layers were washed with brine (50 mL), dried with anhydrous $Na_2SO_4$, filtered, and evaporated under reduced pressure. To the crude acid (approximately 1.5 g), absolute EtOH (40 mL) and concentrated $H_2SO_4$ (5 drops) were added. The reaction mixture was refluxed under argon for 4 h. The solvent was evaporated under reduced pressure, saturated aqueous solution of $NaHCO_3$ (30 mL) was added, and the crude product was extracted with EtOAc (2 × 100 mL). The combined organic phases were washed with brine (50 mL), dried with anhydrous $Na_2SO_4$, filtered, and evaporated under reduced pressure. The compound was purified by column chromatography (MP = EtOAc : n-hexane = 1 : 1).

**Appearance:** white amorphous solid, m = 0.31 g; **Yield:** 21%; **Melting point** 121-123 °C; **ESI-MS $[M-H]^-$:** 247; **HRMS:** (m/z = 247) for $C_{13}H_{11}O_5$: calculated 247.0606, found 247.0604; **$^1$H NMR (400 MHz, acetone-*$d_6$*)** δ **(ppm)** = 1.21 (t, *J* = 7.1 Hz, 3H, $CH_2CH_3$), 3.51 (d, *J* = 0.9 Hz, 2H, $CH_2COO$), 4.12 (q, *J* = 7.1 Hz, 2H, $CH_2CH_3$), 6.76 (d, *J* = 2.6 Hz, 1H, Ar-H), 6.85 (dd, $J_1$ = 8.5 Hz, $J_2$ = 2.3 Hz, 1H, Ar-H), 7.48 (d, *J* = 8.5 Hz, 1H, Ar-H), 7.79 (s, 1H, Ar-H), 9.40 (br s, 1H, OH); **$^{13}$C NMR (100 MHz, acetone-*$d_6$*)** δ **(ppm)** = 14.45, 36.49, 61.23, 103.07, 113.03, 113.89, 119.56, 130.09, 142.59, 156.22, 161.51, 161.83, 170.78; **IR (ATR):** 3555, 3419, 3235, 2984, 1721, 1690, 1614, 1458, 1405, 1372, 1333, 1264, 1183, 1123, 1081, 1017, 965, 915, 862, 830, 775, 735

*Step 2:* **Ethyl 2-(2-oxo-7-(2-oxo-2-phenylethoxy)-2H-chromen-3-yl)acetate**

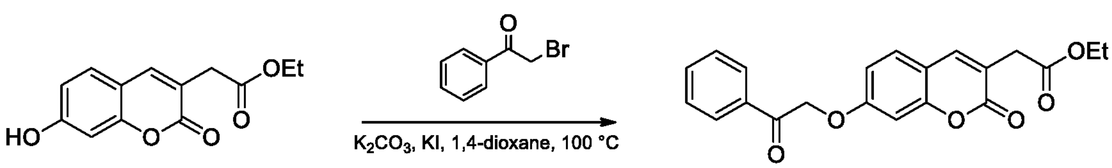


The title compound was prepared from ethyl 2-(7-hydroxy-2-oxo-2*H*-chromen-3-yl)acetate (0.31 g) and 2-bromo-1-phenylethan-1-one following the general procedure A (*O*-alkylation). The compound was purified by column chromatography (MP = EtOAc : n-hexane = 1 : 1).

**Appearance:** pale yellow solid, m = 0.34 g; **Yield:** 76%; **Melting point:** 134-136 °C; **ESI-MS $[M+H]^+$:** 367; **HRMS:** (m/z = 367) for $C_{21}H_{19}O_6$: calculated 367.1182, found 367.1188; **$^1$H NMR (400 MHz, $CDCl_3$)** δ **(ppm)** = 1.25 (t,

$J$ = 7.1 Hz, 3H, $CH_2CH_3$), 3.52 (d, $J$ = 0.8 Hz, 2H, $CH_2COO$), 4.17 (q, $J$ = 7.2 Hz, 2H, $CH_2CH_3$), 5.37 (s, 2H, $COCH_2O$), 6.77 (d, $J$ = 2.5 Hz, 1H, Ar-H), 6.90 (dd, $J_1$ = 8.6 Hz, $J_2$ = 2.5 Hz, 1H, Ar-H), 7.35 (d, $J$ = 8.7 Hz, 1H, Ar-H), 7.47-7.52 (m, 2H, Ar-H), 7.58 (s, 1H, Ar-H), 7.61-7.65 (m, 1H, Ar-H), 7.96-7.87 (m, 2H, Ar-H); **$^{13}$C NMR (100 MHz, $CDCl_3$) δ (ppm)** = 14.06, 35.80, 61.14, 70.48, 101.54, 112.87, 113.36, 119.37, 127.89, 128.67, 128.92, 134.01, 134.16, 141.33, 154.89, 160.57, 161.36, 170.13, 193.08; **IR(ATR):** 2981, 2931, 1722, 1700, 1614, 1563, 1505, 1467, 1446, 1401, 1386, 1368, 1336, 1293, 1232, 1188, 1161, 1142, 1093, 1060, 1014, 989, 964, 910, 864, 809, 775, 762, 729

*Step 3*: **2-(7-Oxo-3-phenyl-7*H*-furo[3,2-*g*]chromen-6-yl)acetic acid**

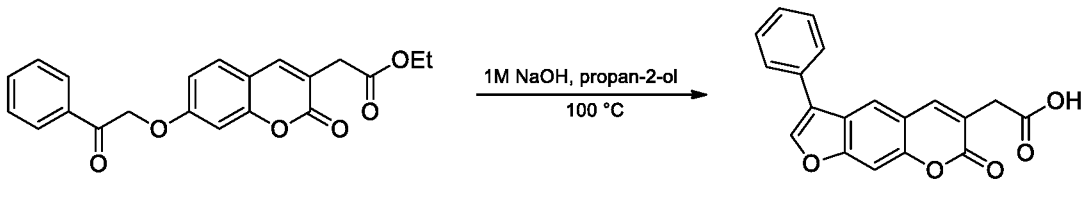


The title compound was prepared from ethyl 2-(2-oxo-7-(2-oxo-2-phenylethoxy)-2*H*-chromen-3-yl)acetate (0.42 g) following the general procedure B (base-catalyzed cyclization). The compound was purified by crystallization from EtOAc.

**Appearance:** pale yellow crystals, m = 0.16 g; **Yield:** 44%; **Melting point** 226-229 °C; **ESI-MS [M+H]$^+$:** 321; **HRMS:** (m/z = 321) for $C_{19}H_{13}O_5$: calculated 321.0763, found 321.0769; **$^1$H NMR (400 MHz, DMSO-$d_6$) δ (ppm)** = 3.51 (s, 2H, $CH_2$), 7.41-7.45 (m, 1H, Ar-H), 7.52-7.56 (m, 2H, Ar-H), 7.78-7.81 (m, 2H, Ar-H), 7.83 (s, 1H, Ar-H), 8.13 (s, 1H, Ar-H), 8.29 (s, 1H, Ar-H), 8.51 (s, 1H, Ar-H), 12.55 (br s, 1H, COOH); **$^{13}$C NMR (100 MHz, acetone-$d_6$) δ (ppm)** = 37.27, 101.25, 118.18, 121.47, 123.28, 123.76, 125.54, 129.22, 129.74, 130.99, 133.01, 143.98, 145.56, 153.67, 158.70, 162.60, 172.44; **IR (ATR):** 3309, 1706, 1638, 1624, 1580, 1460, 1441, 1389, 1369, 1304, 1253, 1234, 1209, 1152, 1091, 1069, 963, 903, 888, 856, 826, 794

EXAMPLE 5

**Synthesis of 6-(aminomethyl)-5-methyl-3-phenyl-7*H*-furo[3,2-*g*]chromen-7-one**

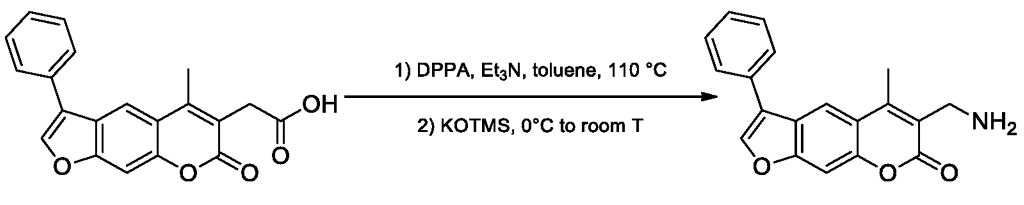


The compound was synthesized following the previously reported modified Curtius reaction (Ma B. et al. Tetrahedron Lett. 2010, 51, 385-386). To a solution of 2-(7-oxo-3-phenyl-7*H*-furo[3,2-*g*]chromen-6-yl)acetic acid (430 mg, 1.29 mmol) in toluene (33 mL), $Et_3N$ (160 mg, 1.55 mmol, 215 μL) and diphenylphosphoryl azide (355 mg, 1.29 mmol, 278 μL) were added. The resulting suspension was stirred at 110 °C for 3 h. A solution of sodium trimethylsilanolate (1 M in THF, 2.6 mL) was added to a cooled (0 °C) reaction mixture and stirred at room temperature for 30 min. The reaction was quenched by the addition of citric acid solution (32 mL, 5% w/w). The organic solvents were evaporated under reduced pressure, followed by the addition of 1 M HCl (40 mL) to the residue. The resulting solution was extracted with $Et_2O$ (2 × 40 mL) and the organic phases were discarded. The aqueous layer was made alkaline with 4 M NaOH and extracted with $CH_2Cl_2$ (3 × 40 mL). The combined organic phases were washed with brine (50 mL), dried with anhydrous $Na_2SO_4$, filtered, and evaporated under reduced pressure. The compound was purified by column chromatography (MP = $CH_2Cl_2$ : MeOH = 9 : 1).

**Appearance:** orange-brown solid, m = 0.24 g; **Yield:** 62%; **Melting point** 123-125 °C; **ESI-MS [M+H]$^+$:** 306; **HRMS:** (m/z = 306) for $C_{19}H_{16}NO_3$: calculated 306.1130, found 306.1121; **$^1$H NMR (400 MHz, $CDCl_3$) δ (ppm)** = 1.79 (br s, 2H, $NH_2$), 2.54 (s, 3H, $CH_3$), 3.86 (s, 2H, $CH_2$), 7.39-7.44 (m, 2H, Ar-H), 7.49-7.53 (m, 2H, Ar-H), 7.59-7.61 (m, 2H, Ar-H), 7.79 (s, 1H, Ar-H), 7.96 (s, 1H, Ar-H); **$^{13}$C NMR (100 MHz, $CDCl_3$) δ (ppm)** = 15.03, 38.87, 99.70, 115.98, 117.12, 122.15, 123.81, 125.09, 127.42, 127.94, 129.15, 130.93, 142.70, 146.94, 150.41, 156.41, 161.70; **IR (ATR):**

3058, 1700, 1627, 1605, 1574, 1466, 1436, 1390, 1334, 1245, 1156, 1111, 1091, 1058, 1030, 900, 868, 843, 779, 760

EXAMPLE 6

**Synthesis of 2,5-dioxopyrrolidin-1-yl 3-(5-methyl-7-oxo-3-phenyl-7*H*-furo[3,2-*g*]chromen-6-yl)propanoate**

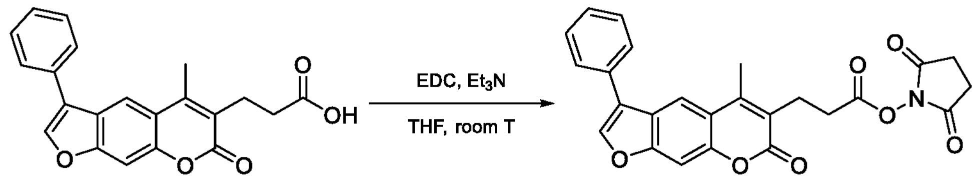


To a solution of 3-(5-methyl-7-oxo-3-phenyl-7*H*-furo[3,2-g]chromen-6-yl)propanoic acid (0.98 g, 2.82 mmol) in dry THF (15 mL), *N*-hydroxysuccinimide (0.41 g, 3.53 mmol) and EDC (0.68 g, 3.53 mmol) were added under argon atmosphere. After five minutes, $Et_3N$ (0.36 g, 3.53 mmol, 0.49 mL) was added. The resulting suspension was stirred at room temperature for 24 h. The solvent was then evaporated under reduced pressure, followed by the addition of EtOAc (50 mL) and citric acid solution (10 %, m/V; 50 mL) to the residue to aid the transfer to a separating funnel. The aqueous phase was further extracted with EtOAc (2 × 20 mL). The combined organic phases were dried with anhydrous $Na_2SO_4$, filtered, and evaporated under reduced pressure. The product was purified by column chromatography (MP = EtOAc : n-hexane = 1 : 1).

**Appearance:** white amorphous solid, m = 0.42 g; **Yield:** 33%; **Melting point:** 203-207 °C; **ESI-MS [M+H]$^+$:** m/z = 446; **HRMS:** (m/z = 446) for $C_{25}H_{20}NO_7$: calculated 446.1240, found 446.1250; **$^1$H NMR (400 MHz, $CDCl_3$)** δ **(ppm)** = 2.56 (s, 3H, Ar-C<u>H</u>$_3$), 2.81 (bs, 4H, succinimide-C<u>H</u>$_2$), 3.01 (t, *J* = 7.5 Hz, 2H, C<u>H</u>$_2$CH$_2$COO), 3.14 (t, *J* = 7.5 Hz, 2H, CH$_2$C<u>H</u>$_2$COO), 7.41-7.46 (m, 1H, Ar-<u>H</u>), 7.50-7.56 (m, 3H, Ar-<u>H</u>), 7.62-7.66 (m, 2H, Ar-<u>H</u>), 7.83 (s, 1H, Ar-<u>H</u>), 8.02 (s, 1H, Ar-<u>H</u>); $^{13}$C **NMR (100 MHz, DMSO-*d*$_6$)** δ **(ppm)** = 15.33, 22.62, 25.37, 28.72, 99.50, 116.65, 116.90, 121.00, 121.23, 122.82, 127.18, 127.82, 129.21, 130.62, 144.33, 148.94,149.87, 155.92, 160.44, 168.22, 170.13; **IR (ATR):** 3608, 2545, 2161, 2026, 1976, 1728, 1707, 1629, 1581, 1393, 1349, 1203, 1160, 1070, 1015, 804, 771, 704, 651

EXAMPLE 7

**Synthesis of 2,5-dioxopyrrolidin-1-yl 2-(3-(4-bromophenyl)-5-methyl-7-oxo-7*H*-furo[3,2-*g*]chromen-6-yl)acetate**

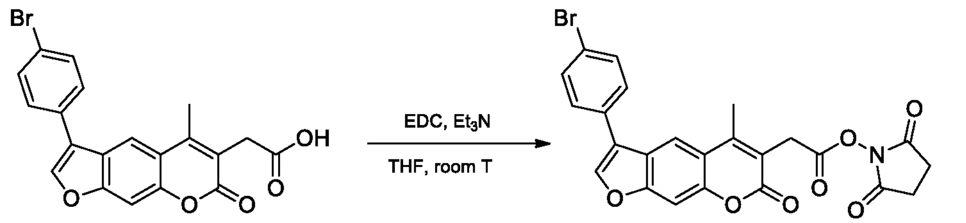


To a solution of 2-(3-(4-bromophenyl)-5-methyl-7-oxo-7*H*-furo[3,2-g]chromen-6-yl)acetic acid (0.45 g, 1.08 mmol) in dry THF (15 mL), *N*-hydroxysuccinimide (0.155 g, 1.35 mmol) and EDC (0.259 g, 1.35 mmol) were added under argon atmosphere. After five minutes, $Et_3N$ (0.137 g, 1.35 mmol, 0.187 mL) was added. The resulting suspension was stirred at room temperature for 24 h. The solvent was then evaporated under reduced pressure, followed by the addition of EtOAc (50 mL) and citric acid solution (10 %, m/V; 50 mL) to the residue to aid the transfer to a separating funnel. The aqueous phase was further extracted with EtOAc (2 × 50 mL). The combined organic phases dried with anhydrous $Na_2SO_4$, filtered, and evaporated under reduced pressure. The product was purified by column chromatography (MP = EtOAc : n-hexane = 2 : 1).

**Appearance:** white amorphous solid, m = 0.083 g; **Yield:** 15%; **Melting point:** 218-223 °C; **ESI-MS [M+H]$^+$:** m/z = 510; **HRMS:** (m/z = 510) for $C_{24}H_{17}NO_7Br$: calculated 510.0188, found 510.0182; **$^1$H NMR (400 MHz, DMSO-*d*$_6$)** δ **(ppm)** = 2.63 (s, 3H, Ar-C<u>H</u>$_3$), 2.80 (bs, 4H, succinimide-C<u>H</u>$_2$), 4.17 (s, 2H, C<u>H</u>$_2$COO), 7.72-7.75 (m, 2H, Ar-<u>H</u>), 7.79-7.83 (m, 2H, Ar-<u>H</u>), 7.88 (s, 1H, Ar-<u>H</u>), 8.26 (s, 1H, Ar-<u>H</u>), 8.56 (s, 1H, Ar-<u>H</u>); **IR (ATR):** 3632, 2545, 2160, 2031, 1976, 1736,

1702, 1199, 1156, 1156, 1066, 1005, 874, 817, 786

EXAMPLE 8

**Synthesis of 2-(3-(4-bromophenyl)-5-methyl-7-oxo-7*H*-furo[3,2-*g*]chromen-6-yl)-*N*-(cyanomethyl)-*N*-methylacetamide**

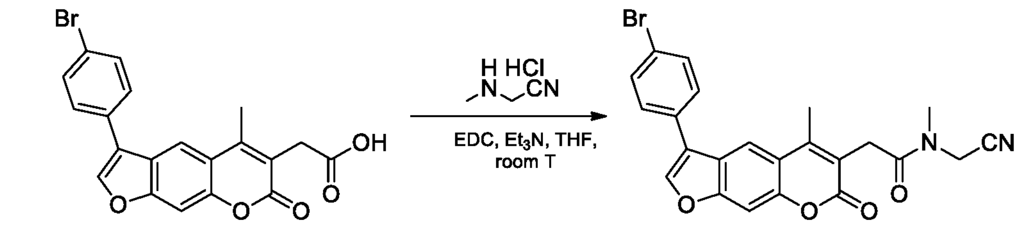


The title compound was prepared from 2-(3-(4-bromophenyl)-5-methyl-7-oxo-7*H*-furo[3,2-g]chromen-6-yl)acetic acid (0.072 g) and *N*-methylaminoacetonitrile hydrochloride following the general procedure C (synthesis of *N*-(cyanomethyl)amides). The compound was purified by column chromatography (MP = EtOAc).

**Appearance:** white amorphous solid, m = 0.017 g; **Yield:** 15%; **Melting point:** 230-234 °C; **ESI-MS [M-H]$^-$:** 463; **HRMS:** (m/z = 463) for $C_{23}H_{16}N_2O_4Br$: calculated 463.0293, found 463.0290; **$^1$H NMR (400 MHz, DMSO-*d$_6$*)** δ **(ppm)** = 2.51 (s, 3H, Ar-C<u>H</u>$_3$), 3.24 (s, 3H, NC<u>H</u>$_3$), 3.83 (s, 2H, C<u>H</u>$_2$CO), 4.43 (s, 2H, C<u>H</u>$_2$CN), 7.72-7.75 (m, 2H, Ar-<u>H</u>), 7.79-7.83 (m, 2H, Ar-<u>H</u>), 7.84 (s, 1H, Ar-<u>H</u>), 8.21 (s, 1H, Ar-<u>H</u>), 8.55 (s, 1H, Ar-<u>H</u>); **IR (ATR):** 3619, 2535, 2160, 2029, 1976, 1693, 1581, 1465, 1392, 1160, 1114, 1071, 880, 861, 834, 792

EXAMPLE 9

**Synthesis of *N*-(cyanomethyl)-2-(5-methyl-7-oxo-3-phenyl-7*H*-furo[3,2-*g*]chromen-6-yl)acetamide**

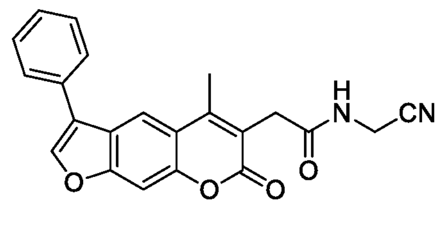

*Step 1:* **Ethyl 2-(4-methyl-2-oxo-7-(2-oxo-2-phenylethoxy)-2*H*-chromen-3-yl)acetate**

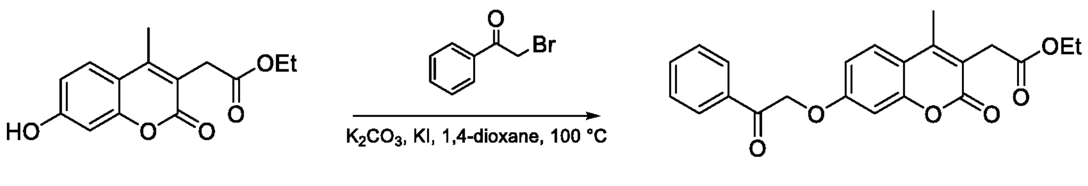


The title compound was prepared from ethyl 2-(7-hydroxy-4-methyl-2-oxo-2*H*-chromen-3-yl)acetate (0.31 g) and 2-bromo-1-phenylethan-1-one according to the general procedure A (*O*-alkylation). The compound was purified by column chromatography (MP = EtOAc : n-hexane = 1 : 1).

**Appearance:** white-yellow amorphous solid, m = 0.210 g; **Yield:** 55%; **Melting point:** 140-142 °C; **ESI-MS [M+H]$^+$: 381; $^1$H NMR (400 MHz, DMSO-*d$_6$*)** δ **(ppm)** = 1.18 (t, *J* = 7.2 Hz, 3H, CH$_2$C<u>H</u>$_3$), 2.39 (s, 3H, Ar-C<u>H</u>$_3$), 3.66 (s, 2H, C<u>H</u>$_2$COO), 4.08 (q, *J* = 7.2 Hz, 2H, COOC<u>H</u>$_2$CH$_3$), 5.76 (s, 2H, COC<u>H</u>$_2$O), 7.05 (dd, *J$_1$* =8.8 Hz, *J$_2$* = 2.6 Hz, 1H, Ar-<u>H</u>), 7.11 (d, *J* = 2.6 Hz, 1H, Ar-<u>H</u>), 7.56-7.62 (m, 2H, Ar-<u>H</u>), 7.69-7.73 (m, 1H, Ar-<u>H</u>), 7.76 (d, *J* = 8.8 Hz, 1H, Ar-<u>H</u>),

8.042-8.07 (m, 2H, Ar-H); **IR (ATR):** 2976, 2931, 1721, 1695, 1610, 1326, 1287, 1229, 1202, 1174, 1097, 1024, 997, 965, 856, 823, 753, 682, 649

*Step 2:* **2-(5-Methyl-7-oxo-3-phenyl-7*H*-furo[3,2-*g*]chromen-6-yl)acetic acid**

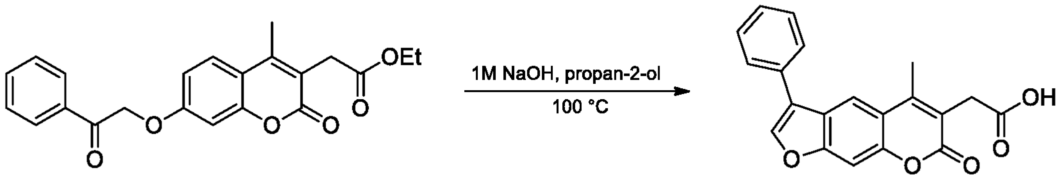


The title compound was prepared from ethyl 2-(4-methyl-2-oxo-7-(2-oxo-2-phenylethoxy)-2*H*-chromen-3-yl)acetate (0.21 g) following the general procedure B (base-catalyzed cyclization). The compound was purified by crystallization from EtOAc.

**Appearance:** yellow-brown crystals, m = 0.120 g; **Yield:** 65%; **Melting point:** 191-196 °C; **ESI-MS $[M+H]^+$:** 335; **$^1$H NMR (400 MHz, DMSO-*$d_6$*) δ (ppm)** = 2.55 (s, 3H, Ar-$CH_3$), 3.66 (s, 2H, $CH_2$COOH), 7.42-7.46 (m, 1H, Ar-H), 7.53-7.57 (m, 2H, Ar-H), 7.82-7.84 (m, 3H, Ar-H), 8.21 (s, 1H, Ar-H), 8.49 (s, 1H, Ar-H), 12.50 (bs, 1H, COOH); **$^{13}$C NMR (100 MHz, DMSO-*$d_6$*) δ (ppm)** = 15.50, 32.86, 99.42, 116.59, 116.62, 118.14, 121.18, 122.77, 127.13, 127.76, 129.14, 130.60, 144.24, 149.20, 149.76, 155.93, 160.64, 171.47; **IR (ATR):** 3635, 2546, 2160, 2030, 1976, 1692, 1630, 1578, 1396, 1335, 1224, 1159, 1109, 1022, 882, 842, 764, 695

*Step 3:* ***N*-(cyanomethyl)-2-(5-methyl-7-oxo-3-phenyl-7*H*-furo[3,2-*g*]chromen-6-yl)acetamide**

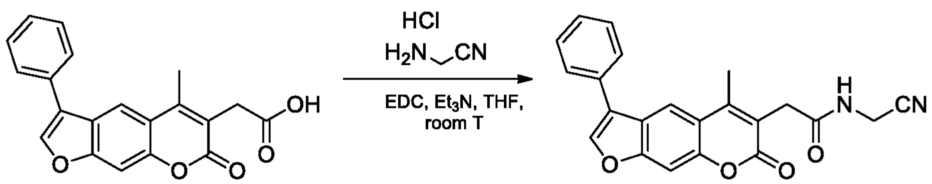


The title compound was prepared from 2-(5-methyl-7-oxo-3-phenyl-7*H*-furo[3,2-g]chromen-6-yl)acetic acid (0.148 g) and aminoacetonitrile hydrochloride following the general procedure C (synthesis of *N*-(cyanomethyl)amides). The compound was purified by column chromatography (MP = EtOAc : n-hexane = 2 : 1).

**Appearance:** white amorphous solid, m = 0.046 g; **Yield:** 28%; **Melting point** 203-209 °C; **ESI-MS $[M-H]^-$:** 371; **HRMS:** (m/z = 371) for $C_{22}H_{15}N_2O_4Br$: calculated 371.1032, found 371.1038; **$^1$H NMR (400 MHz, DMSO-*$d_6$*) δ (ppm)** = 2.53 (s, 3H, Ar-$CH_3$), 3.62 (s, 2H, $CH_2$CO), 4.14 (d, *J*= 5.6 Hz, 2H, NH$CH_2$), 7.41-7.46 (m, 1H, Ar-H), 7.52-7.58 (m, 2H, Ar-H), 7.80-7.86 (m, 3H, Ar-H), 8.22 (s, 1H, Ar-H), 8.49 (s, 1H, Ar-H), 8.70 (t, *J* = 5.6 Hz, 1H, N$HCH_2$); **$^{13}$C NMR (100 MHz, DMSO-*$d_6$*) δ (ppm)** = 15.75, 27.26, 33.83, 99.59, 116.74, 116.96, 117.67, 118.14, 121.34, 122.91, 127.27, 127.91, 129.30, 130.67, 144.37, 150.02, 150.11, 156.10, 160.89, 169.79; **IR (ATR):** 3633, 3278, 3059, 2526, 2160, 2031, 1976, 1704, 1654, 1631, 1538, 1396, 1334, 1160, 1114, 878, 763, 698, 680

EXAMPLE 10

**Synthesis of *N*-(cyanomethyl)-3-(5-methyl-7-oxo-3-phenyl-7*H*-furo[3,2-*g*]chromen-6-yl)propanamide**

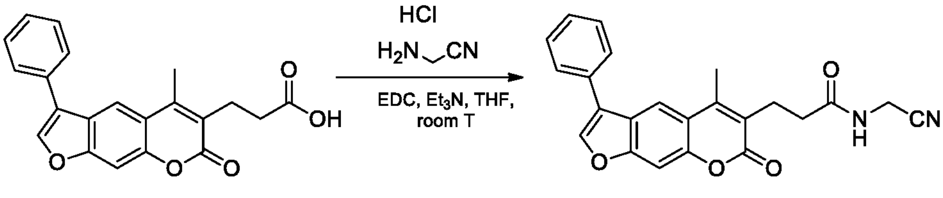

The title compound was prepared from 3-(5-methyl-7-oxo-3-phenyl-7*H*-furo[3,2-*g*]chromen-6-yl)propanoic acid (0.503 g) and aminoacetonitrile hydrochloride following the general procedure C (synthesis of *N*-(cyanomethyl)amides). The compound was purified by column chromatography (MP = EtOAc : n-hexane = 2 : 1).

**Appearance:** red-orange amorphous solid, m = 195 mg; **Yield:** 35%; **Melting point:** 217-223 °C; **ESI-MS $[M+H]^+$: 387; $^1$H NMR (400 MHz, DMSO-$d_6$) δ (ppm)** = 2.38 (t, *J* = 7.5 Hz, 2H, $C\underline{H}_2CH_2CO$), 2.55 (s, 3H, Ar-$C\underline{H}_3$), 2.86 (t, *J* = 7.5 Hz, 2H, $CH_2C\underline{H}_2CO$), 4.10 (d, *J* = 5.5 Hz, 2H, $NHC\underline{H}_2$), 7.42-7.46 (m, 1H, Ar-*H*), 7.52-7.58 (m, 2H, Ar-H), 7.79 (s, 1H, Ar-H), 7.80-7.84 (m, 2H, Ar-H), 8.16 (s, 1H, Ar-*H*), 8.48 (s, 1H, Ar-H), 8.64 (t, *J* = 5.5 Hz, 1H, $N\underline{H}CH_2$); **$^{13}$C NMR (100 MHz, DMSO-$d_6$) δ (ppm)** = 15.15, 23.42, 26.91, 33.37, 99.38, 116.47, 116.94, 116.62, 121.21, 122.49, 122.71, 127.15, 127.79, 129.18, 130.65, 144.23, 147.90, 149.79, 155.78, 160.49, 171.85; **IR (ATR):** 3231, 2809, 2536, 2161, 2032, 1977, 1675, 1629, 1581, 1541, 1396, 1261, 1157, 1092, 1031, 769, 699

EXAMPLE 11

**Synthesis of 5-(5-methyl-7-oxo-3-phenyl-7*H*-furo[3,2-*g*]chromen-6-yl)-1,3,4-oxathiazol-2-one**

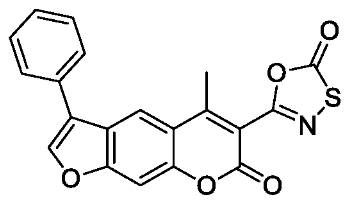

*Step 1:* **Ethyl 7-hydroxy-4-methyl-2-oxo-2*H*-chromene-3-carboxylate**

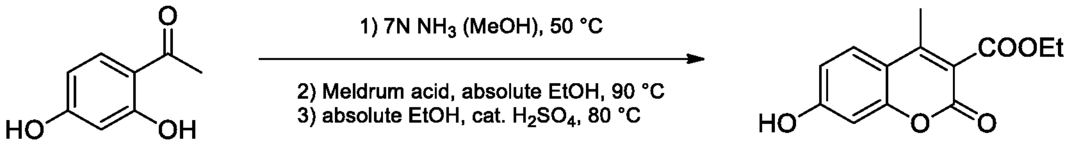


The compound was prepared following the literature procedure (Draoui N. et al. Bioorg. Med. Chem. 2013, 21, 7107-7117). To a 10 mL round-bottomed flask, 2,4-dihydroxyacetophenone (2.00 g, 13.10 mmol) and ammonia solution (7 N $NH_3$ in MeOH, 15.0 mL) were added. The mixture was stirred under argon for 24 h at 50 °C, followed by the evaporation of the solvent under reduced pressure. To the resulting residue, 2,2-dimethyl-1,3-dioxane-4,6-dione (Meldrum acid; 0.35 g, 15.72 mmol) and absolute EtOH (50 mL) were added. The resulting solution was stirred overnight at 90 °C.

The solvent was then evaporated, $CH_2Cl_2$ (50 mL) was added, the solution was transferred to a separating funnel, and extracted with saturated aqueous solution of $NaHCO_3$ (30 mL). The aqueous phase was acidified with concentrated hydrochloric acid and extracted with EtOAc (6× 50 mL). The combined organic phases were washed with brine (100 mL), dried with anhydrous $Na_2SO_4$, filtered, and evaporated under reduced pressure. The compound was crystallized from a mixture of EtOH/$H_2O$, 1/1 (v/v). To a solution of this carboxylic acid (7-hydroxy-4-methyl-2-oxo-2H-chromene-3-carboxylic acid; 0.75 g, 3.41 mmol) in absolute EtOH (15 mL), catalytic amount of concentrated $H_2SO_4$ was added and the resulting solution was refluxed overnight. The solvent was evaporated under reduced pressure and EtOAc (50 mL) was added to the resulting oil. The organic phase was extracted with saturated aqueous solution of $NaHCO_3$ (50 mL), brine (50 mL), dried with anhydrous $Na_2SO_4$, filtered, and evaporated under reduced pressure. The compound was purified by column chromatography (MP = $CH_2Cl_2$ : MeOH = 9 : 1).

**Appearance:** grey-white solid, m = 0.75 g; **Overall yield (for two steps):** 23%; **Melting point:** 130-132 °C; **ESI-MS $[M-H]^-$:** 247; **HRMS:** (m/z = 247) for $C_{13}H_{11}O_5$: calculated 247.0606, found 247.0599; **$^1$H NMR (400 MHz, acetone-$d_6$) δ (ppm)** = 1.34 (t, *J* = 7.1 Hz, 3H, $CH_2C\underline{H}_3$), 2.43 (s, 3H, $C\underline{H}_3$), 4.35 (q, *J* = 7.1 Hz, 2H, $C\underline{H}_2CH_3$), 6.76 (d, *J* = 2.4 Hz, 1H, Ar-H), 6.91 (dd, $J_1$ = 8.8 Hz, $J_2$ = 2.4 Hz, 1H, Ar-H), 7.73 (d, *J* = 8.8 Hz, 1H, Ar-H), 9.68 (br s, 1H, OH); **$^{13}$C NMR (100 MHz, acetone-$d_6$) δ (ppm)** = 15.40, 17.01, 63.11, 104.27, 113.57, 115.21, 119.48, 129.34,152.36,156.75,159.39,163.74,166.71; **IR (ATR):** 3221, 2982,1723,1692, 1600, 1558, 1514, 1444, 1386, 1369, 1328, 1193, 1153, 1079, 1051, 1032, 856

*Step 2:* **Ethyl 4-methyl-2-oxo-7-(2-oxo-2-phenylethoxy)-2H-chromene-3-carboxylate**

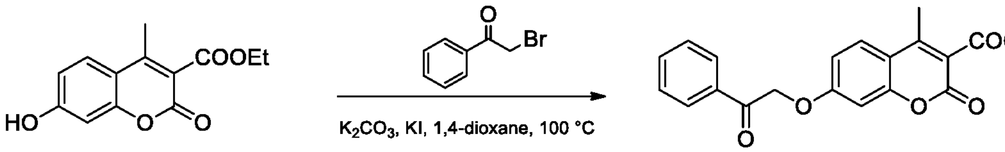


The title compound was prepared from ethyl 7-hydroxy-4-methyl-2-oxo-2*H*-chromene-3-carboxylate (0.21 g) and 2-bromo-1-phenylethan-1-one according to the general procedure A (*O*-alkylation). The compound was purified by column chromatography (MP = EtOAc : n-hexane = 1 : 2).

**Appearance:** pale yellow solid, m = 1.03 g; **Yield:** 82%; **Melting point:** 159-161 °C; **ESI-MS $[M+H]^+$:** 367; **HRMS:** (m/z = 367) for $C_{21}H_{19}O_6$: calculated 367.1182, found 367.1172; **$^1$H NMR (400 MHz, TFA-d) δ (ppm)** = 1.67 (t, *J* = 7.2 Hz, 3H, $CH_2CH_3$), 2.85 (s, 3H, $CH_3$), 4.77 (q, *J* = 7.2 Hz, 2H, $CH_2CH_3$), 5.84 (s, 2H, $COCH_2O$), 7.26 (d, *J* = 2.5 Hz, 1H, Ar-H), 7.44 (dd, $J_1$ = 9.1 Hz, $J_2$ = 2.5 Hz, 1H, Ar-H), 7.77 (t, *J* = 7.9 Hz, 2H, Ar-H), 7.94 (t, *J* = 7.5 Hz, 1H, Ar-H), 8.08 (d, *J* = 9.1 Hz, 1H, Ar-H), 8.29 (dd, $J_1$ = 8.8 Hz, $J_2$ = 1.2 Hz, 2H, Ar-H); **$^{13}$C NMR (100 MHz, TFA-*d*) δ (ppm)** = 5.50, 8.54, 57.83, 63.16, 94.65, 107.14, 108.61, 109.03, 120.84, 121.47, 122.40, 125.84, 129.31, 147.67, 150.85, 155.98, 156.37, 161.56, 191.86; **IR (ATR):** 2971, 1698, 1628, 1606, 1577, 1390, 1364, 1295, 1266, 1199, 1155, 1139, 1062, 810, 760

*Step 3:* **5-Methyl-7-oxo-3-phenyl-7*H*-furo[3,2-*g*]chromene-6-carboxylic acid**

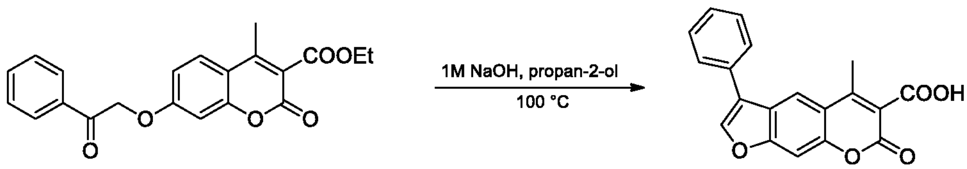


The title compound was prepared from ethyl 4-methyl-2-oxo-7-(2-oxo-2-phenylethoxy)-2*H*-chromene-3-carboxylate (0.92 g) following the general procedure B (base-catalyzed cyclization). The compound was purified by column chromatography (MP = $CH_2Cl_2$ : MeOH : $CH_3COOH$ = 9 : 1 : 0.1).

**Appearance:** yellow-gold solid, m = 0.23 g; **Yield:** 28%; **Melting point:** 203-205 °C; **ESI-MS $[M-H]^-$:** 319; **HRMS:** (m/z = 319) for $C_{19}H_{11}O_5$: calculated 319.0606, found 319.0598; **$^1$H NMR (400 MHz, DMSO-*d*$_6$) δ (ppm)** = 2.47 (s, 3H, $CH_3$), 7.42 (tt, $J_1$ = 7.6 Hz, $J_2$ = 1.8 Hz, 1H, Ar-H), 7.52-7.56 (m, 2H, Ar-H), 7.71 (s, 1H, Ar-H), 7.80 (dd, $J_1$ = 8.4 Hz, $J_2$ = 1.2 Hz, 2H, Ar-H), 8.09 (s, 1H, Ar-H), 8.44 (s, 1H, Ar-H); **$^{13}$C NMR (100 MHz, DMSO-*d*$_6$) δ (ppm)** = 16.19, 99.27, 116.19, 117.07, 121.13, 122.60, 127.14, 127.74 (Ar-C + Ar-CH, confirmed with NMR: $^1$H-$^{13}$C HSQC in $^1$H-$^{13}$C HMBC experiments), 129.15, 130.76, 142.38, 144.02, 149.92, 155.67, 158.57, 167.47; **IR (ATR):** 3448, 3061, 1685, 1561, 1397, 1347, 1269, 1215, 1155, 1111, 1071, 1030, 957, 827, 806, 760

*Step 4:* **Methyl 5-methyl-7-oxo-3-phenyl-7*H*-furo[3,2-*g*]chromene-6-carboxylate**

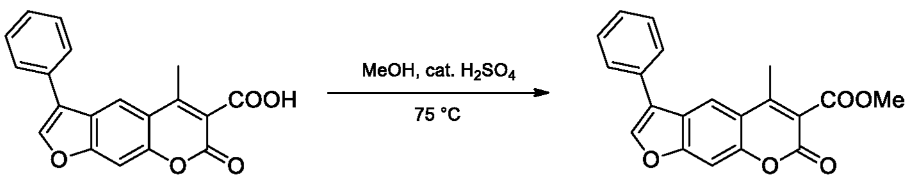


To a solution of 5-methyl-7-oxo-3-phenyl-7*H*-furo[3,2-g]chromene-6-carboxylic acid 0.20 g, 0.60 mmol) in MeOH (50 mL), concentrated $H_2SO_4$ (5 drops) was added. The reaction mixture was stirred at 75 °C overnight. The solvent was then removed under reduced pressure, followed by the addition of saturated aqueous solution of $NaHCO_3$ (50 mL).

The aqueous phase was extracted with EtOAc (3 × 50 mL), the combined organic phases were dried with anhydrous $Na_2SO_4$, filtered, and evaporated under reduced pressure. The compound was purified by column chromatography (MP = EtOAc : n-hexane = 1 : 1).

**Appearance:** pale orange oil, m = 0.12 g; **Yield:** 60%; **$^1$H NMR (400 MHz, $CDCl_3$)** δ **(ppm)** = 2.55 (s, 3H, $CH_3$), 3.98 (s, 3H, $OCH_3$), 7.43-7.47 (m, 1H, Ar-H), 7.50-7.56 (m, 3H, Ar-H), 7.60-7.63 (m, 2H, Ar-H), 7.85 (s, 1H, Ar-H), 8.05 (s, 1H, Ar-H)

*Step 5:* **5-Methyl-7-oxo-3-phenyl-7*H*-furo[3,2-*g*]chromene-6-carboxamide**

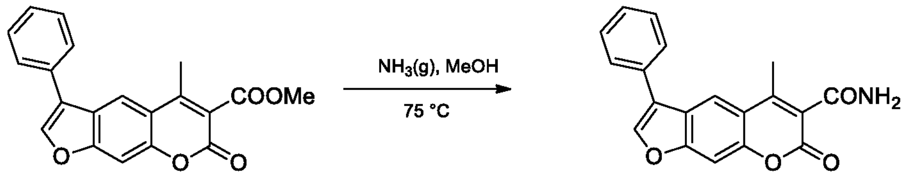


A solution of methyl 5-methyl-7-oxo-3-phenyl-7*H*-furo[3,2-g]chromene-6-carboxylate (0.11 g, 0.33 mmol) in MeOH (40 mL) was agitated under a stream of $NH_{3(g)}$ for 15 min. The reaction vessel was sealed and stirred at 75 °C for 24 h. The solvent was removed under reduced pressure. To the oily residue, EtOAc (100 mL) was added, washed with 0.5 M HCl (50 mL), saturated brine (50 mL), and evaporated under reduced pressure. The compound was purified by crystallization from EtOAc.

**Appearance:** white crystals, m = 0.037 g; **Yield:** 35%; **Melting point:** > 300 °C; **$^1$H NMR (400 MHz, MeOD-*$d_4$*)** δ **(ppm)** = 2.67 (s, 3H, $CH_3$), 7.43-7.47 (m, 1H, Ar-H), 7.54-7.58 (m, 2H, Ar-H), 7.65 (s, 1H, Ar-H), 7.75-7.77 (m, 2H, Ar-H), 8.17 (s, 1H, Ar-H), 8.26 (s, 1H, Ar-H)

*Step 6:* **5-(5-Methyl-7-oxo-3-phenyl-7*H*-furo[3,2-*g*]chromen-6-yl)-1,3,4-oxathiazol-2-one**

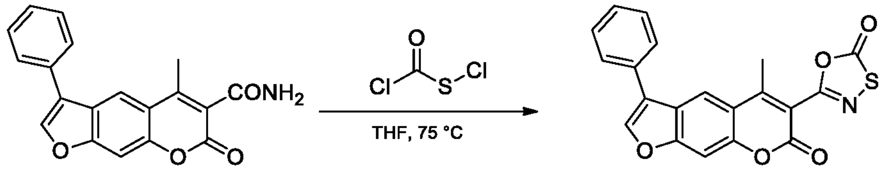


To a suspension of 5-methyl-7-oxo-3-phenyl-7*H*-furo[3,2-g]chromene-6-carboxamide (0.011 g, 0.034 mmol) in THF (20 mL), chlorocarbonylsulfenyl chloride (0.018 g, 0.137 mmol, 0.012 mL) was added at room temperature. The resulting suspension was stirred at 75 °C for 4 h. The solvent was removed under reduced pressure. The compound was subsequently purified by column chromatography (MP = EtOAc : n-hexane = 1 : 1).

**Appearance:** yellow solid, m = 0.006 g; **Yield:** 47%; **Melting point:** 191-194 °C; **$^1$H NMR (400 MHz, $CDCl_3$)** δ **(ppm)** = 2.65 (s, 3H, $CH_3$), 7.45-7.49 (m, 1H, Ar-H), 7.52-7.58 (m, 3H, Ar-H), 7.61-7.64 (m, 2H, Ar-H), 7.88 (s, 1H, Ar-H), 8.13 (s, 1H, Ar-H); **$^{13}$C NMR (100 MHz, $CDCl_3$)** δ **(ppm)** = 17.13, 100.58, 112.75, 115.55, 117.56, 122.44, 124.97, 127.64, 128.35, 129.38, 130.48, 143.57, 151.42, 153.51, 156.15, 157.74, 158.14, 172.88

EXAMPLE 12

**Synthesis of *N*-(5-methyl-7-oxo-3-phenyl-7*H*-furo[3,2-*g*]chromen-6-yl)acrylamide**

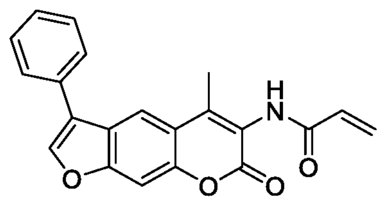

*Step 1:* ***N*-(7-hydroxy-4-methyl-2-oxo-2*H*-chromen-3-yl)acetamide**

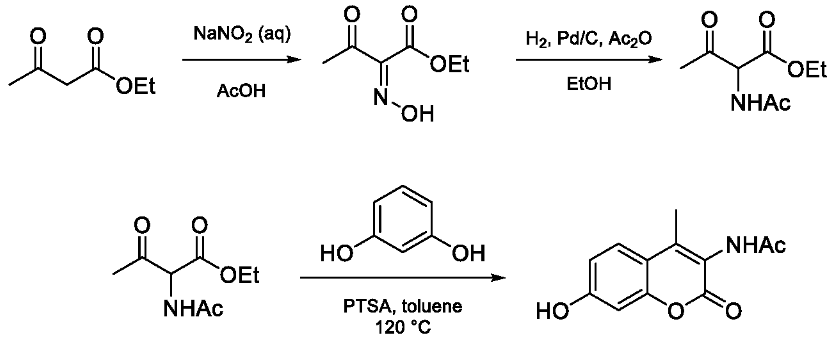


The compound was prepared in five consecutive reactions starting from commercially available ethyl acetoacetate (Seo H.J. et al. J. Med. Chem. 2011, 54, 6305-6318). To a solution of ethyl acetoacetate (10.20 g, 78.38 mmol, 10.00 mL) in acetic acid (12 mL), a solution of $NaNO_2$ (5.95 g, 86.21 mmol) in cold water (16 mL) was added dropwise at -10 °C. The reaction mixture was stirred at -10 °C for 1 h, then water (20 mL) was added and the reaction mixture was stirred for additional 3 h at room temperature. The mixture was extracted with $Et_2O$ (4 × 100 mL), the combined organic phases were washed with saturated aqueous $NaHCO_3$ solution (3 × 100 mL), dried with anhydrous $Na_2SO_4$, filtered, and evaporated under reduced pressure to obtain crude ethyl 2-(hydroxyimino)-3-oxobutanoate as a pale yellow oil (11.12 g) that was used in next step without further purification. Crude ethyl 2-(hydroxyimino)-3-oxobutanoate (10.00 g, 63.63 mmol) was dissolved in EtOH (120 mL) and agitated under a stream of argon for 15 min. Pd/C (0.94 g, 10 wt %) and $AC_2O$ (19.49 g, 190.88 mmol, 18.0 mL) were added and the suspension was stirred overnight under a $H_2$ atmosphere. The palladium was filtered off through a pad of Celite (Celite® 577 fine) and the filtrate was evaporated under reduced pressure. The compound (ethyl 2-acetamido-3-oxobutanoate) was purified by column chromatography (MP = EtOAc : n-hexane = 1 = 1).

**Appearance:** colorless oil, m = 9.77 g; **Yield:** 82% (calculated with respect to 10.00 g of pure ethyl 2-(hydroxyimino)-3-oxobutanoate); **$^1$H NMR (400 MHz, $CDCl_3$) δ (ppm)** = 1.26 (t, *J* = 7.2 Hz, 3H, $CH_2CH_3$), 2.03 (s, 3H, $NHCOCH_3$), 2.34 (s, 3H, $COCH_3$), 4.42 (dq, $J_1$ = 7.2 Hz, $J_2$ = 1.0 Hz, 1H, $CH_2CH_3$), 5.22 (d, *J* = 6.6 Hz, CH), 6.80 (d, *J* = 5.1 Hz, NH)

To a solution of resorcinol (2.04 g, 18.53 mmol) and p-toluenesulfonic acid monohydrate (0.35 g, 1.85 mmol) in dry toluene (120 mL), ethyl 2-acetamido-3-oxobutanoate (8.67 g, 46.32 mmol) was added under argon. The mixture was stirred at 120 °C for 18 h. The solvent was evaporated and the title compound (N-(7-hydroxy-4-methyl-2-oxo-2*H*-chromen-3-yl)acetamide) was purified by crystallization from EtOH.

**Appearance:** white crystals, m = 0.88 g; **Yield:** 20%; **Melting point:** 260-263 °C; **ESI-MS [M-H]$^-$:** 232; **HRMS:** (m/z = 232) for $C_{12}H_{10}NO_4$: calculated 232.0610, found 232.0616; **$^1$H NMR (400 MHz, DMSO-*d*$_6$) δ (ppm)** = 2.04 (s, 3H, $NHCOCH_3$), 2.20 (s, 3H, $CH_3$), 6.67 (d, *J*= 2.2 Hz, 1H, Ar-H), 6.77 (dd, $J_1$ = 8.7 Hz, $J_2$ = 2.3 Hz, 1H, Ar-H), 7.49 (d, *J* = 8.7 Hz, 1H, Ar-H), 9.32 (br s, 1H, NH), 10.26 (br s, 1H, OH); **$^{13}$C NMR (100 MHz, acetone-*d*$_6$) δ (ppm)** = 14.28, 22.51, 101.98, 111.86, 113.19, 117.58, 126.98, 146.66, 152.82, 158.61, 160.59, 168.76; **IR (ATR):** 3320, 3072, 3026, 2964, 2824, 2765, 1713, 1601, 1519, 1474, 1379, 1355, 1337, 1293, 1260, 1248, 1169, 1119, 1070, 846, 800, 771

*Step 2:* ***N*-(4-methyl-2-oxo-7-(2-oxo-2-phenylethoxy)-2*H*-chromen-3-yl)acetamide**

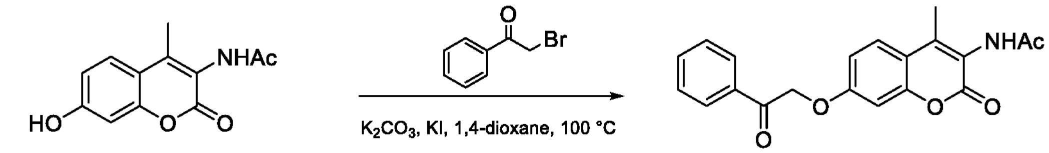


The title compound was prepared from *N*-(7-hydroxy-4-methyl-2-oxo-2*H*-chromen-3-yl)acetamide (0.85 g) and 2-bromo-1-phenylethan-1-one according to the general procedure A (*O*-alkylation). The compound was purified by crystallization from EtOAc.

**Appearance:** white crystals, m = 1.05 g; **Yield:** 82%; **Melting point:** 166-168 °C; **ESI-MS [M+H]$^+$:** 352; **HRMS:** (m/z = 352) for $C_{20}H_{18}NO_5$: calculated 352.1185, found 352.1182; **$^1$H NMR (400 MHz, TFA-*d*) δ (ppm)** = 2.21 (s, 0.2H, CH$_3$), 2.47 (s, 2.8H, CH$_3$), 2.53 (s, 2.8H, CH$_3$), 2.63 (s, 0.2H, CH$_3$), 5.64 (s, 2H, CH$_2$O), 7.08 (d, *J* = 2.4 Hz, 1H, Ar-H), 7.25 (dd $J_1$ = 9.0 Hz, $J_2$ = 2.4 Hz, 1H, Ar-H), 7.58 (d, *J* = 7.8 Hz, 2H, Ar-H), 7.74 (t, *J* = 7.4 Hz, 1H, Ar-H), 7.84 (d, *J*= 7.7 Hz, 1H, Ar-H), 8.09 (d, *J* = 7.7 Hz, 2H, Ar-H); **$^{13}$C NMR (100 MHz, TFA-*d*) δ (ppm)** = 6.67, 13.81, 63.07, 94.81, 107.25, 108.13, 108.82, 120.55, 121.36, 122.26, 125.75, 129.14, 146.43, 147.79, 155.08, 156.34, 170.18, 191.86; **IR (ATR):** 3320, 3257, 3058, 1702, 1691, 1678, 1608, 1515, 1426, 1382, 1360, 1331, 1302, 1257, 1226, 1181, 1163, 1124, 1076, 988, 972, 852, 820, 757

*Step 3:* ***N*-(5-methyl-7-oxo-3-phenyl-7*H*-furo[3,2-*g*]chromen-6-yl)acetamide**

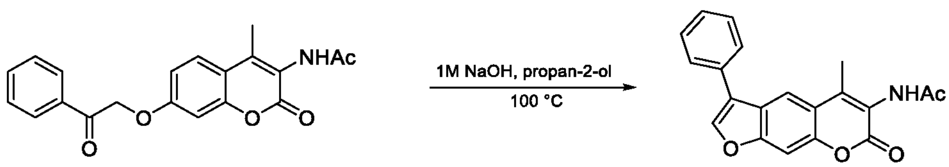


The title compound was prepared from *N*-(4-methyl-2-oxo-7-(2-oxo-2-phenylethoxy)-2*H*-chromen-3-yl)acetamide (0.96 g) following the general procedure B (base-catalyzed cyclization). The compound was purified by crystallization from $CH_2Cl_2$.

**Appearance:** white crystals, m = 0.48 g; **Yield:** 52%; **Melting point:** 241-246 °C; **ESI-MS [M+H]$^+$:** 334; **HRMS:** (m/z = 334) for $C_{20}H_{16}NO_4$: calculated 334.1079, found 334.1082; **$^1$H NMR (400 MHz, DMSO-*d$_6$*) δ (ppm)** = 2.08 (s, 3H, COCH$_3$), 2.40 (s, 3H, CH$_3$), 7.42 (tt, $J_1$ = 7.6 Hz, $J_2$ = 1.6 Hz, 1H, Ar-H), 7.52-7.56 (m, 2H, Ar-H), 7.80-7.82 (m, 3H, Ar-H), 7.15 (s, 1H, Ar-H), 8.47 (s, 1H, Ar-H), 9.57 (br s, 1H, NHCO); **$^{13}$C NMR (100 MHz, DMSO-*d$_6$*) δ (ppm)** = 14.86, 22.55, 99.59, 116.55, 116.90, 119.69, 121.18, 122.99, 127.17, 127.80, 129.16, 130.57, 144.38, 146.42, 149.23, 155.97, 158.21, 168.79; **IR (ATR):** 3343, 3113, 1713, 1682, 1625, 1579, 1517, 1458, 1439, 1388, 1347, 1244, 1145, 1069

*Step 4*: **6-Amino-5-methyl-3-phenyl-7*H*-furo[3,2-*g*]chromen-7-one**

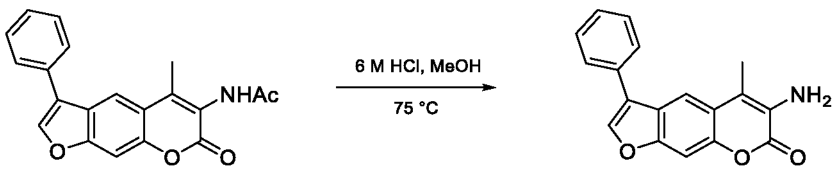


A suspension of *N*-(5-methyl-7-oxo-3-phenyl-7*H*-furo[3,2-*g*]chromen-6-yl)acetamide (0.45 g, 1.35 mmol) in MeOH (35 mL) and 6 M HCl (27 mL) was heated to 75 °C for 14 h. The reaction mixture was allowed to cool to room temperature and was then neutralized with saturated aqueous $NaHCO_3$ solution, to pH~10. Methanol was evaporated under reduced pressure and the aqueous residue was extracted with EtOAc (3 × 50 mL), the combined organic phases were further washed with brine (50 mL), dried with anhydrous $Na_2SO_4$, filtered, and evaporated under reduced pressure. The compound was purified by crystallization from EtOH.

**Appearance:** white crystals, m = 0.20 g; **Yield:** 52%; **Melting point:** 236-239 °C; **ESI-MS [M+H]$^+$:** 292; **HRMS:** (m/z = 292) for $C_{18}H_{14}NO_3$: calculated 292.0974, found 292.0982; **$^1$H NMR (400 MHz, CDCl$_3$) δ (ppm)** = 2.30 (s, 3H,

$CH_3$), 4.06 (br s, 2H, $NH_2$), 7.42 (tt, $J_1$ = 7.2 Hz, $J_2$ = 1.2 Hz, 1H, Ar-H), 7.47 (s, 1H, Ar-H), 7.52 (t, $J$ = 7.6 Hz, 2H, Ar-H), 7.64-7.66 (m, 2H, Ar-H), 7.79 (s, 1H, Ar-H), 7.80 (s, 1H, Ar-H); **$^{13}$C NMR (100 MHz, $CDCl_3$) δ (ppm)** = 12.34, 99.67, 112.96, 118.42, 119.75, 122.02, 123.92, 127.49, 127.51, 127.77, 129.12, 131.45, 142.40, 146.95, 154.26, 159.17; **IR (ATR):** 3472, 3365, 3097, 1694, 1619, 1602, 1569, 1468, 1437, 1285, 1270, 1157, 1093, 850, 829, 758

*Step 5:* ***N*-(5-methyl-7-oxo-3-phenyl-7*H*-furo[3,2-*g*]chromen-6-yl)acrylamide**

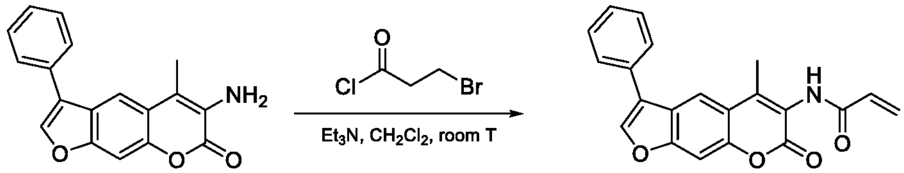


To a solution of 6-amino-5-methyl-3-phenyl-7*H*-furo[3,2-g]chromen-7-one (0.095 g, 0.33 mmol) in $CH_2Cl_2$ (10 mL), $Et_3N$ (0.135 mg, 1.32 mmol, 0.185 mL) and 3-bromopropionyl chloride (0.13 g, 0.72 mmol, 0.076 mL) were added under argon. The reaction mixture was stirred overnight at room temperature. Then, $CH_2Cl_2$ (20 mL) was added to the mixture and extracted with 1 M HCl (30 mL), water (30 mL), saturated aqueous solution of $NaHCO_3$ (30 mL), and brine (30 mL), and dried with anhydrous $Na_2SO_4$, filtered, and evaporated under reduced pressure. The compound was purified by column chromatography (MP = EtOAc : n-hexane = 1 : 1).

**Appearance:** white amorphous solid, m = 0.043 g; **Yield:** 38%; **Melting point:** 232-234 °C; **ESI-MS [M+H]$^+$:** 346; **HRMS:** (m/z = 346) for $C_{21}H_{16}NO_4$: calculated 346.1079, found 346.1085; **$^1$H NMR (400 MHz, DMSO-*$d_6$*) δ (ppm)** = 2.43 (s, 3H, $CH_3$), 5.80 (dd, $J_1$ = 10.1 Hz, $J_2$ = 1.8 Hz, 1H, acrylamide), 6.26 (dd, $J_1$ = 17.1 Hz, $J_2$ = 1.9 Hz, 1H, acrylamide), 6.53 (dd, $J_1$ = 17.1 Hz, $J_2$ = 10.2 Hz, 1H, acrylamide), 7.41-7.47 (m, 1H, Ar-H), 7.52-7.59 (m, 2H, Ar-H), 7.83-7.86 (m, 2H, Ar-H), 7.87 (s, 1H, Ar-H), 8.21 (s, 1H, Ar-H), 8.51 (s, 1H, Ar-H), 9.82 (br s, 1H, NH); **$^{13}$C NMR (100 MHz, DMSO-*$d_6$*) δ (ppm)** = 15.02, 99.70, 116.54, 117.04, 119.35, 121.22, 123.09, 127.21, 127.34, 127.83, 129.19, 130.56, 130.75, 144.48, 146.60, 149.30, 156.07, 158.09, 163.76; **IR (ATR):** = 3133, 2988, 1717, 1652, 1625, 1606, 1578, 1538, 1437, 1403, 1288, 1344, 1319, 1250, 1228, 1159, 1128, 1081, 1016, 976, 953, 936, 911, 879, 814, 791, 753

EXAMPLE 13

**Synthesis of *N*-((5-methyl-7-oxo-3-phenyl-7*H*-furo[3,2-*g*]chromen-6-yl)methyl)acrylamide**

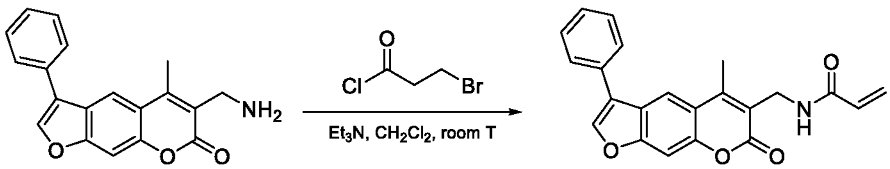


To a solution of 6-(aminomethyl)-5-methyl-3-phenyl-7*H*-furo[3,2-g]chromen-7-one (0.043 g, 0.14 mmol) in $CH_2Cl_2$ (10 mL), $Et_3N$ (0.057 g, 0.56 mmol, 0.078 mL) and 3-bromopropionyl chloride (0.053 g, 0.31 mmol, 0.031 mL) were added under argon. The reaction mixture was stirred overnight at room temperature. Then, $CH_2Cl_2$ (20 mL) was added to the mixture and extracted with 1 M HCl (30 mL), water (30 mL), saturated aqueous solution of $NaHCO_3$ (30 mL), and brine (30 mL), and dried with anhydrous $Na_2SO_4$, filtered, and evaporated under reduced pressure. The compound was purified by column chromatography (MP = EtOAc : n-hexane = 1 : 1) and subsequent crystallization from EtOAc.

**Appearance:** white amorphous solid, m = 0.019 g; **Yield:** 38%; **Melting point:** 191.5-194 °C; **ESI-MS [M+H]$^+$:** 360; **HRMS:** (m/z = 360) for $C_{22}H_{18}NO_4$: calculated 360.1236, found 360.1231; **$^1$H NMR (400 MHz, DMSO-*$d_6$*) δ (ppm)** = 2.65 (s, 3H, $CH_3$), 4.36 (d, $J$ = 5.6 Hz, 2H, $CH_2NH$), 5.58 (dd, $J_1$ = 10.1 Hz, $J_2$ = 2.3 Hz, 1H, acrylamide), 6.10 (dd, $J_1$ = 17.1 Hz, $J_2$ = 2.3 Hz, 1H, acrylamide), 6.26 (dd, $J_1$ = 17.1 Hz, $J_2$ = 10.1 Hz, 1H, acrylamide), 7.42-7.46 (m, 1H, Ar-H), 7.53-7.58 (m, 2H, Ar-H), 7.82-7.84 (m, 3H, Ar-H), 8.23 (s, 1H, Ar-H), 8.38 (t, $J$ = 5.2 Hz, 1H, NH), 8.49 (s, 1H, Ar-H); **$^{13}$C NMR (100 MHz, DMSO-*$d_6$*) δ (ppm)** = 15.00, 17.49, 99.66, 116.60, 116.96, 119.58, 121.21, 123.05, 124.84 127.20, 127.82, 129.18, 130.57, 140.38, 144.45, 146.40, 149.27, 156.01, 158.19, 163.99; **IR (ATR):** = 3269, 3057, 1704,

1648, 1622, 1573, 1529, 1469, 1434, 1393, 1330, 1304, 1236, 1156, 1110, 1095, 1072, 1030, 987, 953, 882, 845, 795, 760, 743

EXAMPLE 14

**Synthesis of -2-cyano-*N*-(5-methyl-7-oxo-3-phenyl-7*H*-furo[3,2-*g*]chromen 6-yl)acetamide**

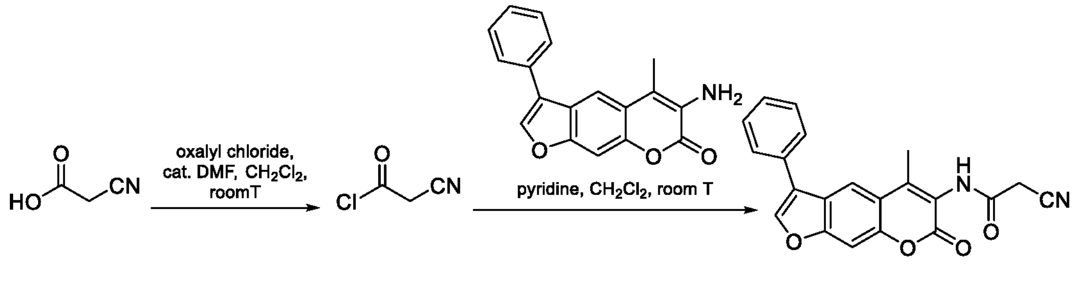


Oxalyl chloride (144 mg, 1.14 mmol, 0.10 mL) and a catalytic amount of DMF were added to a solution of cyanoacetic acid (48 mg, 0.57 mmol) in $CH_2Cl_2$ (10 mL). The reaction mixture was stirred at room temperature for 1 h. The solvent was evaporated and the residue dried under vacuum to obtain an acyl chloride that was dissolved in $CH_2Cl_2$ (5 mL) and added dropwise to the solution of 6-amino-5-methyl-3-phenyl-7*H*-furo[3,2-g]chromen-7-one (110 mg, 0.38 mmol) and pyridine (120 mg, 1.52 mmol, 0.125 mL) in $CH_2Cl_2$ (5 mL). The reaction mixture was stirred at room temperature for 1 h. Then, $CH_2Cl_2$ (30 mL) was added and washed subsequently with 1 M HCl (2 × 30 mL), water (30 mL), saturated aqueous solution of $NaHCO_3$ (30 mL), brine (30 mL), dried with anhydrous $Na_2SO_4$, filtered, and evaporated under reduced pressure. The compound was purified by column chromatography (MP = $CH_2Cl_2$ : MeOH = 20 : 1).

**Appearance:** white amorphous solid, m = 0.099 g; **Yield:** 48%; **Melting point:** 232-236 °C; **ESI-MS [M+H]$^+$:** 359; **HRMS:** (m/z = 359) for $C_{21}H_{15}N_2O_4$: calculated 359.1032, found 359.1041; **$^1$H NMR (400 MHz, DMSO-*d$_6$*)** δ **(ppm)** = 2.46 (s, 3H, C<u>H</u>$_3$), 4.00 (s, 2H, C<u>H</u>$_2$), 7.44 (tt, $J_1$ = 7.4 Hz, $J_2$ = 1.4 Hz, 1H, Ar-<u>H</u>), 7.53-7.58 (m, 2H, Ar-<u>H</u>), 7.83-7.85 (m, 2H, Ar-<u>H</u>), 7.87 (s, 1H, Ar-<u>H</u>), 8.22 (s, 1H, Ar-<u>H</u>), 8.52 (s, 1H, Ar-<u>H</u>), 10.00 (br s, 1H, N<u>H</u>); **$^{13}$C NMR (100 MHz, DMSO-*d$_6$*)** δ **(ppm)** = 14.79, 25.53, 99.72, 115.83, 116.34, 117.20, 118.79, 121.21, 123.11, 127.21, 127.84, 129.18,130.52, 144.52, 147.28, 149.34, 156.17, 157.80, 161.89; **IR(ATR):** =3239, 3091, 2943, 2257, 1710, 1670, 1630, 1605, 1577, 1530, 1468, 1437, 1406, 1393, 1343, 1292, 1272, 1241, 1159, 1132, 1092, 1069, 1018, 974, 949, 926, 903, 969, 821, 799, 781, 759, 741

EXAMPLE 15

**Synthesis of 2-cyano-*N*-((5-methyl-7-oxo-3-phenyl-7*H*-furo[3,2-*g*]chromen-6-yl)methyl)acetamide**

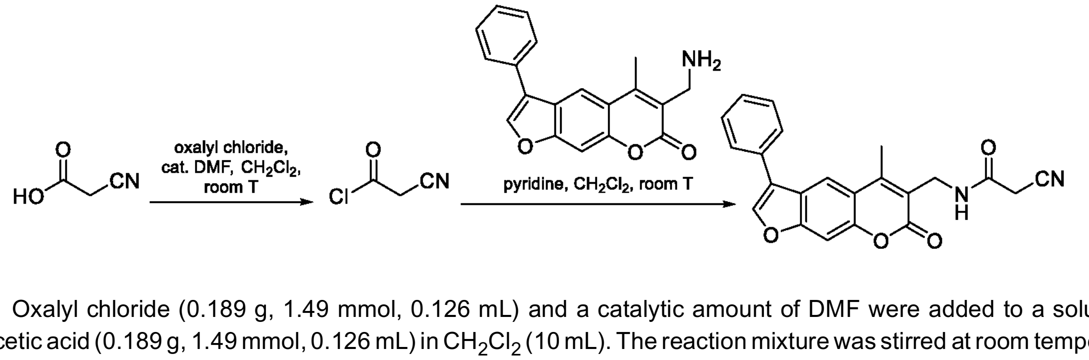


Oxalyl chloride (0.189 g, 1.49 mmol, 0.126 mL) and a catalytic amount of DMF were added to a solution of cyanoacetic acid (0.189 g, 1.49 mmol, 0.126 mL) in $CH_2Cl_2$ (10 mL). The reaction mixture was stirred at room temperature for 1 h. The solvent was evaporated and the residue dried under vacuum to obtain an acyl chloride that was dissolved in $CH_2Cl_2$ (5 mL) and added dropwise to the solution of 6-(aminomethyl)-5-methyl-3-phenyl-7*H*-furo[3,2-g]chromen-7-one (0.10 g, 0.38 mmol) and pyridine (0.104 g, 1.32 mmol, 0.106 mL) in $CH_2Cl_2$ (5 mL). The reaction mixture was stirred at room temperature for 1 h. Then, $CH_2Cl_2$ (30 mL) was added and washed subsequently with 1 M HCl (2 × 30 mL),

water (30 mL), saturated aqueous solution of $NaHCO_3$ (30 mL), brine (30 mL), dried with anhydrous $Na_2SO_4$, filtered, and evaporated under reduced pressure. The compound was purified by column chromatography (MP = EtOAc : n-hexane = 1 :1).

**Appearance:** white solid, m = 0.014 g; **Yield:** 11%; **Melting point:** 219.5-220 °C; **ESI-MS [M+H]+:** 373; **HRMS:** (m/z = 373) for $C_{22}H_{17}N_2O_4$: calculated 373.1188, found 373.1178; **$^1$H NMR (400 MHz, DMSO-*d6*) δ (ppm)** = 2.65 (s, 3H, CH3), 3.65 (s, 2H, $CH_2CN$), 4.31 (d, *J*= 5.2 Hz, 2H, $CH_2NH$), 7.43-7.47 (m, 1H, Ar-H), 7.54-7.58 (m, 2H, Ar-H), 7.82-7.85 (m, 3H, Ar-H), 8.24 (s, 1H, Ar-H), 8.50 (s, 1H, Ar-H), 8.58 (t, *J*= 5.2 Hz, 1H, NH); $^{13}$C **NMR (100 MHz, DMSO-*d6*) δ (ppm)** = 15.33, 25.05, 35.93, 99.58, 116.12, 116.66, 117.09, 119.88, 121.25, 122.92, 127.19, 127.83, 129.19, 130.53, 144.42, 150.06, 150.36, 156.20, 160.51, 162.01; **IR (ATR):** = 3324, 3052, 2915, 1673, 1624, 1575, 1548, 1468, 1397, 1351, 1327, 1235, 1214, 1163, 1115, 1090, 1030, 940, 881, 796, 766

EXAMPLE 16

**Synthesis of (*E*)-*N*-((5-methyl-7-oxo-3-phenyl-7*H*-furo[3,2-*g*]chromen-6-yl)methyl)but-2-enamide**

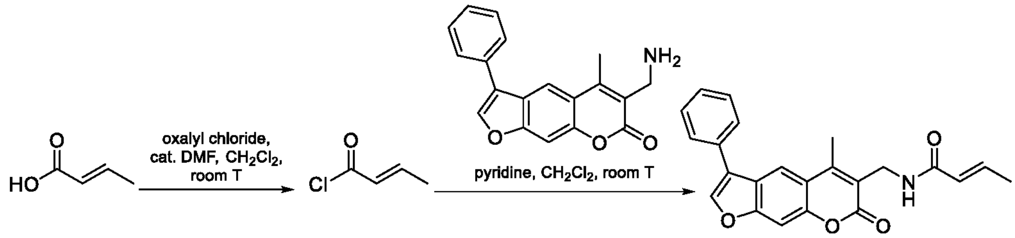


To a solution of trans-2-butenoic acid (133 mg, 1.54 mmol) in $CH_2Cl_2$ (5 mL), a catalytic amount of DMF and oxalyl chloride (401 mg, 3.09 mmol, 0.268 mL) were added at room temperature. The reaction mixture was stirred for 2 h. The solvent was evaporated and the residue was dried under vacuum. The crude acyl chloride was dissolved in $CH_2Cl_2$ (10 mL) and added dropwise to the cooled (0 °C) solution of 6-(aminomethyl)-5-methyl-3-phenyl-7*H*-furo[3,2-g] chromen-7-one (140 mg, 0.46 mmol) and pyridine (0.326 g, 4.12 mmol, 0.332 mL) in $CH_2Cl_2$ (10 mL). The reaction mixture was stirred at room temperature overnight. Then, $CH_2Cl_2$ (30 mL) was added, the solution transferred to a separating funnel, and washed subsequently with 1 M HCl (3 × 20 mL), water (30 mL), saturated aqueous $NaHCO_3$ solution (30 mL), and brine (30 mL), and dried with anhydrous $Na_2SO_4$, and evaporated under reduced pressure. The compound was purified by column chromatography (MP = EtOAc : n-hexane = 1 : 1).

**Appearance:** white amorphous solid, m = 0.030 g; **Yield:** 17%; **Melting point:** 219.0-222.5 °C; **ESI-MS [M+H]+:** 374; **HRMS:** (m/z = 374) for $C_{23}H_{20}NO_4$: calculated 374.1392, found 374.1402; **$^1$H NMR (400 MHz, DMSO-*d6*) δ (ppm)** = 1.76 (d, $J_1$ = 6.9 Hz, $J_2$ = 1.6 Hz, 3H, $CHCH_3$), 2.63 (s, 3H, $CH_3$), 4.33 (d, *J*= 5.2 Hz, 2H, $CH_2NH$), 5.93 (dd, $J_1$ = 15.3 Hz, $J_1$ = 1.7 Hz, 1H, $CH=CHCH_3$), 6.21 (dq, $J_1$ = 14.8 Hz, $J_2$ = 6.8 Hz, 1H, $CH=CHCH_3$), 7.42-7.46 (m, 1H, Ar-H), 7.53-7.58 (m, 2H, Ar-H), 7.81-7.84 (m, 3H, Ar-H), 8.15 (t, *J* = 5.0 Hz, 1H, NH), 8.23 (s, 1H, Ar-H), 8.49 (s, 1H, Ar-H); **$^{13}$C NMR (100 MHz, DMSO-*d6*) δ (ppm)** = 15.32, 17.32, 35.35, 99.52, 116.81, 116.94, 120.56, 121.26, 122.87, 125.43, 127.20, 127.84, 129.21, 130.58,138.05, 144.36, 150.07, 150.11,156.15, 160.60, 164.68; **IR (ATR):** = 2922, 2853, 1705, 1628, 1577, 1530, 1464, 1439, 1391, 1340, 1260, 1156, 1083, 1025, 969, 839, 796, 760

EXAMPLE 17

**Synthesis of (*E*)-*N*-(5-methyl-7-oxo-3-phenyl-7*H*-furo[3,2-*g*]chromen-6-yl)but-2-enamide**

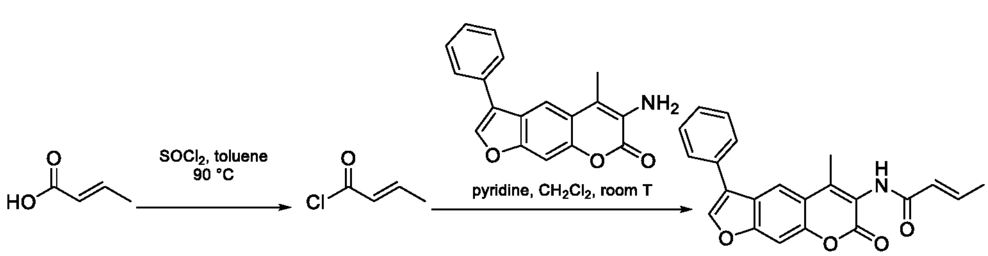

To a solution of trans-2-butenoic acid (67 mg, 0.78 mmol) in toluene (5 mL), thionyl chloride (414 mg, 3.48 mmol, 0.253 mL) was added at room temperature. The reaction mixture was stirred at 90 °C for 2 h. The solvent was evaporated and the residue dried under vacuum to obtain the crude acyl chloride that was dissolved in $CH_2Cl_2$ (10 mL) and added drop-wise to the cooled (0 °C) solution of compound 6-amino-5-methyl-3-phenyl-7*H*-furo[3,2-g]chromen-7-one (150 mg, 0.52 mmol) and pyridine (163g, 2.06 mmol, 0.166 mL) in $CH_2Cl_2$ (10 mL). The reaction mixture was stirred at room temperature overnight. Then, $CH_2Cl_2$ (30 mL) was added, the solution was transferred to a separating funnel, and washed subsequently with 1 M HCl (3 × 20 mL), water (30 mL), saturated aqueous $NaHCO_3$ solution (30 mL), and brine (30 mL), and dried with anhydrous $Na_2SO_4$, and evaporated under reduced pressure. The compound was purified by column chromatography (MP = EtOAc : n-hexane = 1 : 2) and subsequent crystallization from EtOAc.

**Appearance:** white crystals, m = 0.040 g; **Yield:** 21%; **Melting point:** 236.5-238.0 °C; **ESI-MS [M+H]⁺:** 360; **HRMS:** (m/z = 360) for $C_{22}H_{18}NO_4$: calculated 360.1236, found 360.1246; **$^1$H NMR (400 MHz, DMSO-*d*$_6$) δ (ppm)** = 1.88 (d, *J* = 6.8 Hz, 3H, $CHCH_3$), 2.41 (s, 3H, $CH_3$), 6.21 (d, *J* = 16.0 Hz, 1H, $CH{=}CHCH_3$), 6.21 (dq, $J_1$ = 14.4 Hz, $J_2$ = 6.8 Hz, 1H, $CH{=}CHCH_3$), 7.42-7.46 (m, 1H, Ar-H), 7.53-7.57 (m, 2H, Ar-H), 7.83-7.85 (m, 2H, Ar-H), 7.86 (s, 1H, Ar-H), 8.20 (s, 1H, Ar-H), 8.51 (s, 1H, Ar-H), 9.59 (br s, 1H, NH); **$^{13}$C NMR (100 MHz, DMSO-*d*$_6$) δ (ppm)** = 15.00, 17.49, 99.66, 116.60, 116.96, 119.58, 121.21, 123.05, 124.84, 127.20, 127.82, 129.18, 130.57, 140.38, 144.45, 146.40, 149.27, 156.01, 158.19, 163.99; **IR (ATR):** = 3253, 1713, 1669, 1638, 1579, 1512, 1469, 1437, 1390, 1339, 1243, 1196, 1158, 1130, 1088, 1067, 1026, 971, 934, 902, 836, 822, 760, 735

EXAMPLE 18

**Synthesis of 3-(5,9-dimethyl-7-oxo-3-phenyl-7*H*-furo[3,2-*g*]chromen-6-yl)propanoic acid**

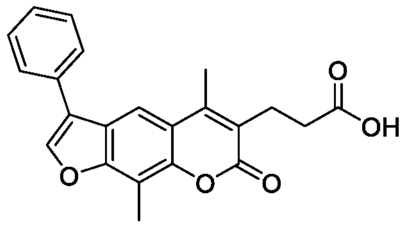

*Step 1:* **Ethyl 3-(7-hydroxy-4,8-dimethyl-2-oxo-2H-chromen-3-yl)propanoate**

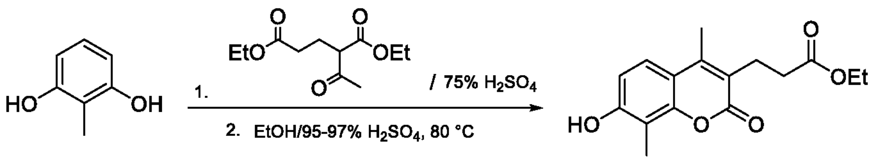


To the solution of 2-methylresorcinol (12.90 g, 104.00 mmol) in sulfuric (VI) acid (75%, 60 mL), diethyl 2-acetylglutarate (26.80 g, 116.00 mmol, 25.00 mL) was added dropwise. The reaction mixture was stirred at room temperature for 18 h. After the reaction was complete, the solution was poured onto crushed ice. The solid that formed was filtered off, washed with water and dried. Anhydrous ethanol (60 mL) and the catalytic amount of concentrated sulfuric (VI) acid (0.50 mL) were added to the dried solid. The reaction mixture was stirred at 80 °C overnight. The solvent was evaporated, and saturated aqueous solution of $NaHCO_3$ (50 mL) was added to the resulting residue, and further extracted with EtOAc (2 × 100 mL). The combined organic phases were dried over $Na_2SO_4$, filtered, and evaporated under reduced pressure.

**Appearance:** white amorphous solid, m = 21.2 g; **Yield:** 70%; **Melting point:** 153-157 °C; **ESI-MS [M+H]⁺:** 291; **HRMS:** (m/z = 291) for $C_{16}H_{18}O_5$: calculated 291.1229, found 291.1232; **$^1$H NMR (400 MHz, DMSO-*d*$_6$) δ (ppm)** = 1.15 (t, *J* = 7.0 Hz, 3H, $CH_2CH_3$), 2.14 (s, 3H, Ar-$CH_3$), 2.35 (s, 3H, Ar-$CH_3$), 2.46 (t, *J* = 7.6 Hz, 2H, $CH_2COO$), 2.78 (t, *J* = 7.6 Hz, 2H, Ar-$CH_2$), 4.03 (q, *J* = 7.0 Hz, 2H, $COOCH_2$), 6.84 (d, *J* = 8.8 Hz, 1H, Ar-H), 7.45 (d, *J* = 8.8, 1H, Ar-H), 10.31 (bs, 1H, OH); **IR (ATR):** 3230, 2986, 2958, 2364, 2344, 1868, 1723, 1675, 1606, 1577, 1507, 1465, 1378, 1363, 1297, 1255, 1192, 1143, 1102, 1052, 1027

*Step 2:* **Ethyl 3-(4,8-dimethyl-2-oxo-7-(2-oxo-2-phenylethoxy)-2H-chromen-3-yl)propanoate**

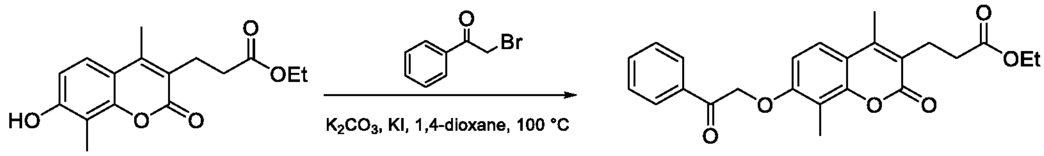


The title compound was prepared from ethyl 3-(7-hydroxy-4,8-dimethyl-2-oxo-2*H*-chromen-3-yl)propanoate (1.02 g) and 2-bromo-1-phenylethan-1-one according to the general procedure A (*O*-alkylation). The compound was purified by crystallization from EtOAc.

**Appearance:** pale yellow crystals, m = 0.871 g; **Yield:** 61%; **Melting point:** 183-187 °C; **ESI-MS $[M+Li]^+$:** 415; **ESI-MS $[M+Na]^+$:** 431; **$^1$H NMR (400 MHz, DMSO-*d*$_6$)** $\delta$ **(ppm)** = 1.15 (t, *J* = 7.2 Hz, 3H, $CH_2CH_3$), 2.28 (s, 3H, Ar-$CH_3$), 2.39 (s, 3H, Ar-$CH_3$), 2.48 (t, *J* = 7.6 Hz, 2H, $CH_2COO$), 2.82 (t, *J* = 7.6 Hz, 2H, Ar-$CH_2$), 4.04 (q, *J* = 7.2 Hz, 2H, $COOCH_2$), 5.78 (s, 2H, $COCH_2O$), 6.99 (d, *J* = 8.8, 1H, Ar-H), 7.56-7.61 (m, 3H, Ar-H), 7.67-7.73 (m, 1H, Ar-H). 8.02-8.04 (m, 2H, Ar-H); **IR (ATR):** 3397, 3063, 2979, 2907, 2363, 2344, 1724, 1709, 1636,1601, 1500, 1474, 1500, 1449, 1378, 1358, 1329, 1284, 1252, 1225, 1206, 1174, 1133, 1069, 1000

*Step 3:* **3-(5,9-Dimethyl-7-oxo-3-phenyl-7*H*-furo[3,2-*g*]chromen-6-yl)propanoic acid**

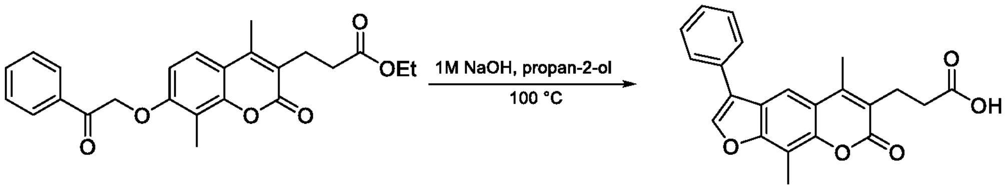


The title compound was prepared from ethyl 3-(4,8-dimethyl-2-oxo-7-(2-oxo-2-phenylethoxy)-2*H*-chromen-3-yl)propanoate (0.254 g) following the general procedure B (base-catalyzed cyclization). The compound was purified by crystallization from EtOAc.

**Appearance:** pale brown crystals, m = 0.15 g; **Yield:** 67%; **Melting point:** 189-193 °C; **ESI-MS $[M+H]^+$:** 363; **HRMS:** (m/z = 363) for $C_{22}H_{19}O_5$: calculated 363.1236, found 363.1246; **$^1$H NMR (400 MHz, DMSO-*d*$_6$)** $\delta$ **(ppm)** = 2.43 (t, *J* = 7.9 Hz, 2H, $CH_2COOH$), 2.53 (s, 3H, Ar-$CH_3$), 2.55 (s, 3H, Ar-$CH_3$), 2.85 (t, *J* = 7.9 Hz, 2H, Ar-$CH_2$), 7.40-7.45 (m, 1H, Ar-H), 7.52-7.56 (m, 2H, Ar-H), 7.79-7.82 (m, 2H, Ar-H), 8.01 (s, 1H, Ar-H), 8.48 (s, 1H, Ar-H), 12.22 (bs, 1H, COOH); **$^{13}$C NMR (100 MHz, DMSO-*d*$_6$)** $\delta$ **(ppm)** = 8.15, 15.16,22.96,32.29, 108.34, 113.44, 116.76, 121.44, 121.67, 121.97, 127.07, 127.69, 129.13, 130.79, 143.96, 147.44, 148.10, 154.75, 160.43, 173.59; **IR (ATR):** 3854, 3421, 3094, 2363, 2344, 1742, 1717, 1661, 1591, 1566, 1419, 1387, 1340, 1300, 1198, 11169, 1105, 1132, 1027

**Biological procedures**

**Biological example 1: Preliminary evaluation of inhibition of the β5i subunit of human immunoproteasome**

Appropriate stock solutions (mM concentration) of the test compounds were prepared in DMSO. Subsequent dilutions of the compounds, as well as the immunoproteasome and the substrate were prepared in buffered solution (50 mM Tris-HCl (pH 8.0), 0.5 mM EDTA, 0.01% SDS). To a 96-well black microtiter plate, human immunoproteasome (final concentration, 0.25 μg/mL) (Boston Biochem, Inc., Cambridge, MA, USA) was added, followed by either test compounds (10 μM final concentration) or buffered solution (with and without the DMSO), which accounted for the control group. Microtiter plate was covered with aluminum foil and incubated at 37 °C for 30 minutes with medium agitation. The reaction was initiated by the addition of β5- and β5i-selective substrate Suc-LLVY-AMC (25 μM final concentration). The reaction progress was recorded on the BioTek Synergy HT microplate reader by monitoring fluorescence at 460 nm ($\lambda_{ex}$ = 360 nm) every 5 minutes for 120 min at 37 °C. At least two independent experiments were performed. The initial linear ranges (between 20th and 60th minute) were used to calculate the slope of the line (*k*) by dividing the difference of fluorescence intensities (F) with the time difference (*t*) (*Equation 1*). The residual activities in percentages were calculated from the

slopes obtained (*Equation* 2). The calculations were performed in Microsoft Office Excel 2007.

$$k = \Delta F / \Delta t \qquad (Equation\ 1)$$

*k* slope of the line

*F* fluorescence intensity

*t* time (min)

$$Residual\ activity\ in\ \% = k_t / k_{nt} * 100\ \% \qquad (Equation\ 2)$$

$k_t$ slope of the line for the treated enzyme

$k_{nt}$ slope of the line for the non-treated enzyme

**Biological example 2: Evaluation of reversible inhibitors of the β5i subunit of human immunoproteasome**

The test compounds from the preliminary inhibitory assay with residual activities equal to or lower than 60% were characterized further by determining the mode of inhibition (competitive, non-competitive, uncompetitive, mixed inhibition). The assays were performed as described above. Five or more different concentrations of the test compounds ranging from 0.1 μM to 100 μM were used to determine the kinetic parameters at four different substrate concentrations (Suc-LLVY-AMC ranging from 12.5 μM to 125 μM). Final concentration of the immunoproteasome remained the same as before (0.25 μg/mL). The results obtained were analyzed as outlined above (see *Equations 1* and 2), and two plots were derived using Microsoft Office Excel 2007:

**[0146]** - 1/v plotted against inhibitor concentration (Dixon plot; v- velocity, equal to the slope of the line k),

**[0147]** - [S]/v plotted against different concentrations of inhibitor ([S] equals substrate concentration).

The mode of inhibition was estimated from the relative positions of the lines obtained. This data was, together with the *k* values, was further used to calculate the $K_i$ values (GraphPad Prism 5.01).

**Biological example 3: Evaluation of irreversible inhibitors of the β5i subunit of human immunoproteasome**

The test compounds with time-dependent inhibitory activities were treated and evaluated as irreversible inhibitors. The assay was performed as described in the 44 section *Preliminary evaluation of inhibition of the β5i subunit of human immunoproteasome.* At least seven different concentrations of the test compounds ranging from 0.001 μM to 50 μM were used. The final concentrations of the substrate Suc-LLVY-AMC and the immunoproteasome were 25 μM and 0.25 μg/mL, respectively. At least two independent experiments were performed. The increases in fluorescence intensity between 20$^{th}$ and 60$^{th}$ minute and appropriate test compounds concentrations were analyzed with GraphPad Prism 5.01 to determine the $IC_{50}$ values.

**Table 1.** Inhibitory potencies of reversible psoralen-based inhibitors against the β5i subunit of the human immunoproteasome. The following compounds are not part of the invention.

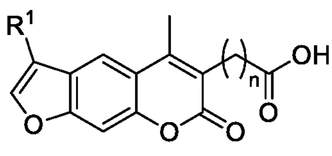

| n | $^1R$ | RA (%) or $K_i$ (μM) | n | $^1R$ | RA (%) or $K_i$ (μM) |
|---|---|---|---|---|---|
| 1 | Me | 88% | 2 | Me | 94% |
| 1 | *t*-Bu | 85% | 2 | *t*-Bu | 83% |

(continued)

| n | $^{1}$R | RA (%) or $K_i$ ($\mu$M) | n | $^{1}$R | RA (%) or $K_i$ ($\mu$M) |
|---|---|---|---|---|---|
| 1 | 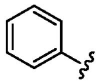 | 2.4[b] | 2 | 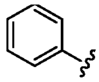 | 1.6[b] |
| 1 | 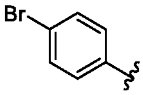 | 12.7[b] | 2 | 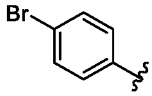 | 137[b] |
| 1 | 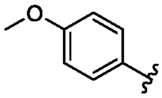 | 8.2[b] | 2 | 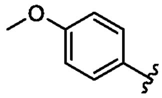 | 84% |
| 1 | 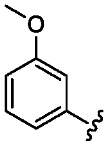 | 7.0[b] | 2 | 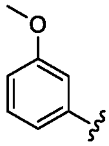 | 29.6[b] |
| 1 | 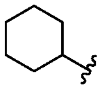 | 198[b] | 2 | 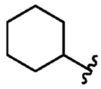 | 102% |
| 1 | 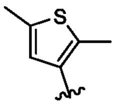 | 36.5[b] | 2 | 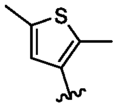 | 52.3[b] |
| 1 | 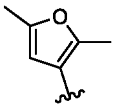 | 351[b] | 2 | 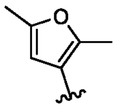 | 100% |
| 1 | 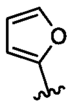 | 89.3[b] | 2 | 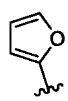 | 115.6[b] |
| 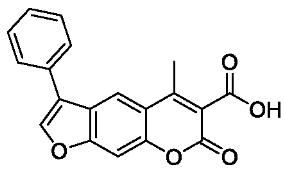 | | 82% | | | |
| 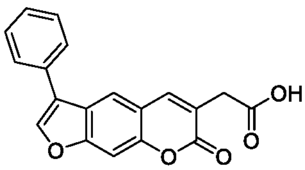 | | 3.8[b] | | | |
| 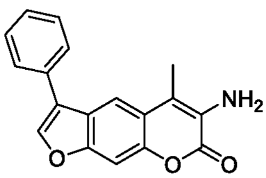 | | 88% | | | |

(continued)

| n | 1R | RA (%) or $K_i$ (μM) | n | 1R | RA (%) or $K_i$ (μM) |
|---|---|---|---|---|---|
| 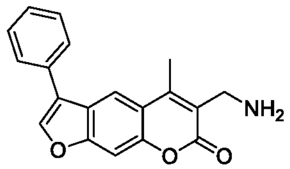 | | 39.6[b] | | | |
| [b]Competitive inhibitors | | | | | |

**Table 2.** Inhibitory potencies of irreversible psoralene-based inhibitors against the β5i subunit of the human immunoproteasome.

| Compound | $IC_{50}$ (μM) |
|---|---|
| 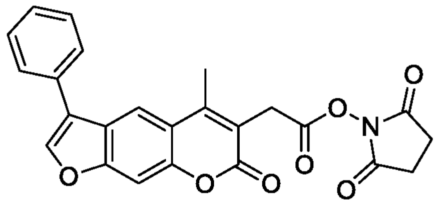 | 6.4 |
| 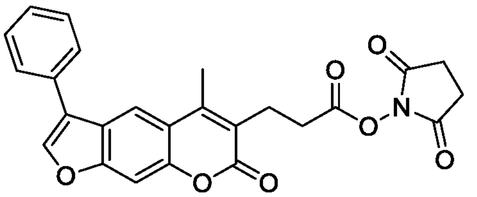 | 0.090 |
| 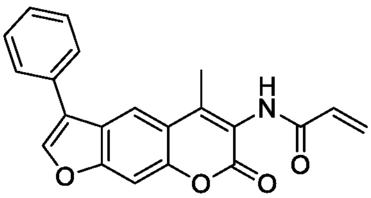 | 20.5 |

## Claims

**1.** A compound having general Formula (I):

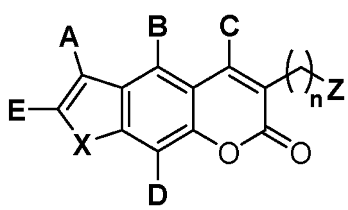

**(I)**

wherein:

**n**: 0-4

**X**: O
Z: is

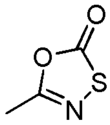,

CHO, $CONHCH_2CN$, $CON(Me)CH_2CN$, $NHCOCH_2CN$, $N(Me)COCH_2CN$, $NHCH_2CN$, $N(Me)CH_2CN$, NHCO-COMe, N(Me)C(=O)COMe, $NHCOCH=CH_2$, $N(Me)COCH=CH_2$, $SO_2F$,

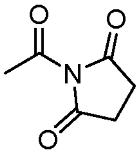,

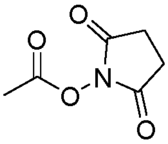, 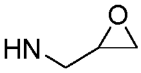, 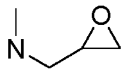, 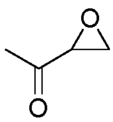 or 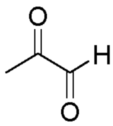

**A**: is t-Bu, phenyl, furan, thiophene, pyrrole, imidazole or oxazole
**B**: is H or Me
**C**: is H or Me
**D**: is H or Me
**E:** is H or Me

or a pharmaceutically acceptable salt, hydrate or solvate thereof.

**2.** The compound of claim 1, wherein Z is

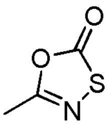, 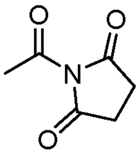

or

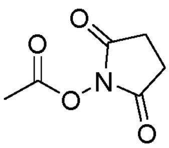.

**3.** The compound of claim 1, wherein Z is

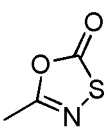 or 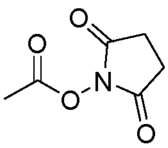.

**4.** The compound of any one of claims 1 to 3, wherein A is phenyl, B is H, C is Me, D is H, E is H, n is 0 and Z is

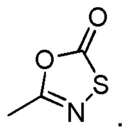

**5.** The compound of claim 1, wherein A is phenyl, B is H, C is Me, D is H, E is H, n is 1 or 2 and Z is

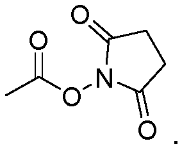

**6.** The compound of any one of claims 1 to 5, which is in the form of a pure diastereoisomer or as a mixture of diastereomers, or in the form of a pure enantiomer or as a mixture of enantiomers.

**7.** The compound of any one of claims 1 to 6 for use as a medicament in human or mammal therapy.

**8.** The compound of any one of claims 1 to 6 for use in the treatment of a disease that is related with increased activity of the immunoproteasome; the disease being selected from the group consisting of autoimmune diseases, malignant diseases, and neurodegenerative disorders.

**9.** A pharmaceutical composition comprising a therapeutically effective amount of at least one compound of any one of claims 1 to 6 and a pharmaceutical acceptable excipient.

## Patentansprüche

**1.** Verbindung der allgemeinen Formel (I):

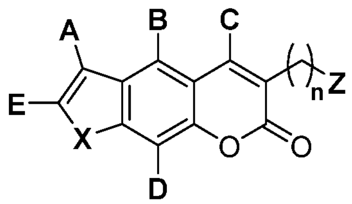

**(I)**

wobei:

**n**: 0-4
**X**: O
**Z**:

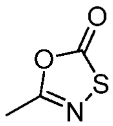,

CHO, $CONHCH_2CN$, $CON(Me)CH_2CN$, $NHCOCH_2CN$, $N(Me)COCH_2CN$, $NHCH_2CN$, $N(Me)CH_2CN$, NH-COCOMe, N(Me)C(=O)COMe, $NHCOCH=CH_2$, $N(Me)COCH=CH_2$, $SO_2F$,

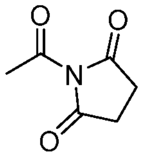,

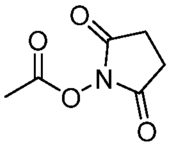, 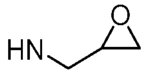, 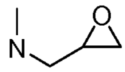, 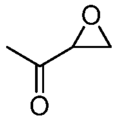 oder 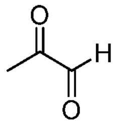

ist
**A**: t-Bu, Phenyl, Furan, Thiophen, Pyrrol, Imidazol oder Oxazol ist
**B**: H oder Me ist
**C**: H oder Me ist
**D**: H oder Me ist
**E**: H oder Me ist

oder ein pharmazeutisch verträgliches Salz, Hydrat oder Solvat davon.

**2.** Verbindung nach Anspruch 1, wobei Z

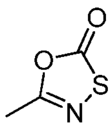, 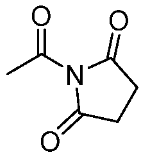

oder

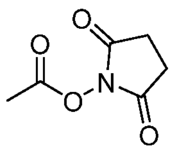

ist.

**3.** Verbindung nach Anspruch 1, wobei Z

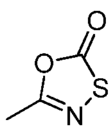 oder 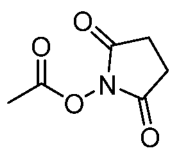

ist.

**4.** Verbindung nach einem der Ansprüche 1 bis 3, wobei A Phenyl ist, B H ist, C Me ist, D H ist, E H ist, n 0 ist und Z

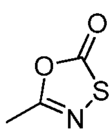

ist.

**5.** Verbindung nach Anspruch 1, wobei A Phenyl ist, B H ist, C Me ist, D H ist, E H ist, n 1 oder 2 ist und Z

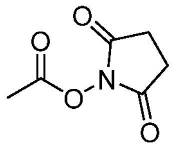

ist.

**6.** Verbindung nach einem der Ansprüche 1 bis 5, welche in Form eines reinen Diastereoisomers oder als ein Gemisch aus Diastereomeren, oder in Form eines reinen Enantiomers oder als ein Gemisch aus Enantiomeren vorliegt.

**7.** Verbindung nach einem der Ansprüche 1 bis 6 zur Verwendung als ein Arzneimittel bei der Therapie von Menschen und Säugetieren.

**8.** Verbindung nach einem der Ansprüche 1 bis 6 zur Verwendung bei der Behandlung einer Erkrankung, die mit einer erhöhten Aktivität des Immunproteasoms in Zusammenhang steht; wobei die Erkrankung aus der Gruppe bestehend aus Autoimmunerkrankungen, bösartigen Erkrankungen und neurodegenerativen Erkrankungen ausgewählt ist.

**9.** Pharmazeutische Zusammensetzung umfassend eine therapeutisch wirksame Menge von mindestens einer Verbindung nach einem der Ansprüche 1 bis 6 und einem pharmazeutisch verträglichen Hilfsstoff.

## Revendications

**1.** Composé avec la formule suivante (I):

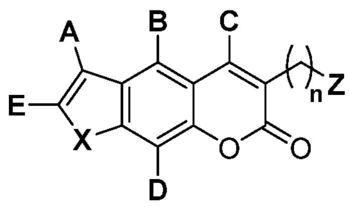

**(I)**

dans laquelle,

**n**: 0-4
**X**: O
**Z:** est

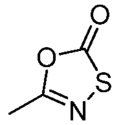,

CHO, $CONHCH_2CN$, $CON(Me)CH_2CN$, $NHCOCH_2CN$, $N(Me)COCH_2CN$, $NHCH_2CN$, $N(Me)CH_2CN$, NHCO-COMe, NMeC=OCOMe, $NHCOCH=CH_2$, $N(Me)COCH=CH_2$, $SO_2F$,

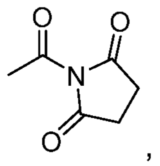,

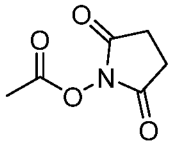, 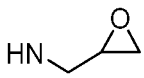, 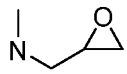, 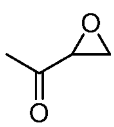 ou 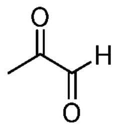

**A**: est t-Bu, phényle, furanne, thiophène, pyrrole, imidazole ou oxazole
**B**: est H ou Me
**C**: est H ou Me
**D**: est H ou Me
**E:** est H ou Me

ou un sel pharmaceutiquement acceptable, hydrate ou solvate de celui-ci.

**2.** Composé selon la revendication 1, dans lequel Z est

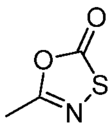, 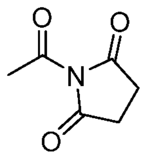

ou

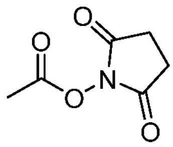.

**3.** Composé selon la revendication 1, dans lequel Z est

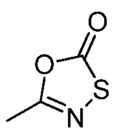

ou

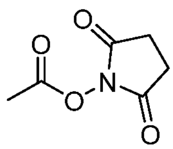.

**4.** Compose selon l'une quelconque des revendications 1 à 3, dans lequel A est phényle, B est H, C est Me, D est H, E est H, n est 0 et Z est

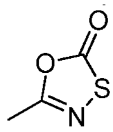

.

5. Compose selon la revendication 1, dans lequel A est phényle, B est H, C est Me, D est H, E est H, n est 1 ou 2 et Z est

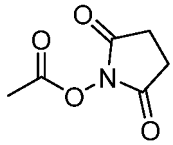

.

6. Compose selon l'une quelconque des revendications 1 à 5, qui est sous forme d'un diastéréoisomère pur ou en tant que mélange de diastéréoisomères, ou sous forme d'un énantiomère pur ou en tant que mélange d'énantiomères.

7. Composé selon l'une quelconque des revendications 1 à 6 pour l'utilisation en tant qu'un médicament utile pour une thérapie chez l'homme ou un mammifère.

8. Composé selon l'une quelconque des revendications 1 à 6 pour l'utilisation dans le traitement d'une maladie qui est liée à une activité accrue de l'immunoprotéasome ; la maladie étant choisie dans le groupe constitué par les maladies auto-immunes, les maladies malignes et les troubles neurodégénératifs.

9. Composition pharmaceutique comprenant une quantité thérapeutiquement efficace d'au moins un composé selon l'une quelconque des revendications 1 à 6 et un excipient pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6346534 B1


### Non-patent literature cited in the description


- **KISH-TRIER E. et al.** *Ann. Rev. Biophys.,* 2013, vol. 49, 29-49
- **ARENDT C.S. et al.** *Proc. Natl. Acad. Sci.,* 1997, vol. 94, 7156-7161
- **GROETTRUP M. et al.** *Nat. Rev. Immunol.,* 2010, vol. 10, 73-78
- **LEE W.** *Curr. Med. Chem.,* 2011, vol. 11, 2923-2930
- **KRAUSE S. et al.** *Ann. Rheum. Dis.,* 2006, vol. 65, 1021-1027
- **BASLER M. et al.** *J. Immunol.,* 2010, vol. 185, 634-641
- **VISEKRUNA A.** *J. Clin. Invest.,* 2006, vol. 116, 3195-320
- **SCHMIDT N.** *Gut,* 2010, vol. 59, 896-906
- **MUCHAMUEL T.** *Nat. Med.,* 2009, vol. 15, 781-787
- **FRANKLAND-SEARBY S.** *Biochim. Biophys. Acta,* 2012, vol. 1825, 64-76
- **BELLAVISTA E.** *Curr. Pharm. Design,* 2013, vol. 19, 702-718
- **KUHN D.J.** *Blood,* 2009, vol. 113, 4667-4676
- **SINGH A.V.** *Br. J. Haematol.,* 2011, vol. 152, 155-163
- **DIAZ-HERNANDEZ M.** *J. Neurosci.,* 2003, vol. 23, 11653-11661
- **MISHTO M.** *Neurobiol. Aging,* 2006, vol. 27, 54-66
- **HUBER E.M.** *Angew. Chem. Int. Ed.,* 2012, vol. 51, 2-15
- **MILLER Z.** *Curr. Pharm. Design.,* 2013, vol. 19, 4140-4151
- **ICHIKAWA H.T.** *Arthritis Rheum.,* 2012, vol. 64, 493-503
- **KOVÁČOVÁ S. et al.** *Chem. Pap.,* 2010, vol. 64, 806-811
- **MA B. et al.** *Tetrahedron Lett.,* 2010, vol. 51, 385-386
- **DRAOUI N. et al.** *Bioorg. Med. Chem.,* 2013, vol. 21, 7107-7117
- **SEO H.J. et al.** *J. Med. Chem.,* 2011, vol. 54, 6305-6318